(19) Europäisches Patentamt
European Patent Office
Office européen des brevets

(11) **EP 4 678 732 A1**

(12) **EUROPEAN PATENT APPLICATION**
published in accordance with Art. 153(4) EPC

(43) Date of publication:
**14.01.2026 Bulletin 2026/03**

(21) Application number: **24769892.1**

(22) Date of filing: **10.03.2024**

(51) International Patent Classification (IPC):
*C12N 1/20* (2026.01)      *A61K 35/741* (2015.01)
*A61P 3/00* (2006.01)      *A61P 3/10* (2006.01)
*A61P 3/04* (2006.01)

(52) Cooperative Patent Classification (CPC):
**A23L 33/135; A61K 31/155; A61K 35/741;
A61K 45/06; A61P 1/16; A61P 3/00; A61P 3/04;
A61P 3/06; A61P 3/08; A61P 3/10; A61P 9/00;
A61P 9/10; A61P 9/12; C12N 1/20;** C12R 2001/01

(86) International application number:
**PCT/CN2024/080883**

(87) International publication number:
**WO 2024/188199 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.03.2023 CN 202310234900**

(71) Applicant: **Moon (Guangzhou) Biotech Co., Ltd.
Guangzhou, Guangdong 510535 (CN)**

(72) Inventors:
• **HUANG, Baojia
Guangzhou, Guangdong 510535 (CN)**
• **KONG, Ping
Guangzhou, Guangdong 510535 (CN)**
• **XIAN, Yibo
Guangzhou, Guangdong 510535 (CN)**

(74) Representative: **Prüfer & Partner mbB
Patentanwälte · Rechtsanwälte
Sohnckestraße 12
81479 München (DE)**

Remarks:
The complete document including Reference
Table(s) and the Sequence Listing(s) can be
downloaded from the EPO website

(54) **ISOLATED CHRISTENSENELLA, COMPOSITION COMPRISING SAME, AND USE**

(57)     The present invention provides an isolated Christensenella, a composition containing same, and a use thereof. The present invention can be used for treating, preventing, or alleviating obesity, obesity-induced metabolic disorders, diabetes, inflammation, liver and kidney diseases, liver diseases, cardiovascular and cerebrovascular diseases and the like.

EP 4 678 732 A1

**Description**

**FIELD OF THE INVENTION**

The present application relates to the field of microbiology, and more specifically to a novel isolated *Christensenella sp.,* a composition comprising same, and a use thereof.

**BACKGROUND OF THE INVENTION**

*Christensenella* is a genus of intestinal commensal bacteria that has been identified in recent years, and comprises an extremely limited number of species, with only six species based on a query on the LPSN, all of which were discovered in recent years.

The screening of novel species within *Christensenella* has faced significant challenges because they primarily present in the intestinal tracts of human or animal, are strictly anaerobic microorganisms, have extremely high requirements for nutrition and culture environment, and have long growth cycles. Furthermore, during the validation of drug efficacy, the anaerobic nature of the bacteria of this genus makes it difficult to verify efficacy through *in vitro* cell experiments; during *in vivo* animal experiments, the bacteria of this genus are difficult to maintain a stable number of viable bacteria due to the difficulty of cultivation and the high degree of anaerobicity, and there is a problem of insufficient reproducibility. All these *in vitro* and *in vivo* related technical difficulties have limited the discovery and application of new species of *Christensenella.*

The current prevalence of metabolic diseases such as obesity, diabetes, hypertension, hyperlipidemia, and fatty liver disease is increasing, and studies have also indicated that these diseases are related to dysbiosis of the microbial community. In many populations, certain bacteria of the *Christensenella* are associated with lower body mass index. *Christensenella minuta (C. mintua)* is one of the most studied species of the *Christensenella,* yet at the same time there are reports suggesting that it causes inflammation in the treatment of obesity, such as acute appendicitis. However, avoiding inflammation during obesity treatment is a pressing need and a critical consideration for the regulatory approval of clinical weight loss drugs.

Mixtures of multiple microorganisms are commonly used in the prior art for the treatment of metabolic and obesity-related diseases or disorders, such as probiotics or fecal microbiota transplantation (FMT). However, mixtures of multiple microorganisms make the mechanism more complex, the interactions between various microorganisms remain poorly researches, and the use of mixtures of microorganisms can disrupt the homeostasis of the intestinal microbiota. In comparison, single bacterium exert less impact on the homeostasis of the intestinal microbiota. Moreover, the use of mixtures of microorganisms requires additional consideration of whether the individual microorganism has potential mutual influences on activity, and it is also not clear whether the therapeutic or preventive effects on diseases result from synergistic actions of the mixtures of microorganisms or from the action of a certain single bacterium.

The number of microorganism resources is extremely large, and screening therefrom for novel species that can be effectively used to treat or prevent metabolic diseases such as obesity, diabetes, hypertension, hyperlipidemia, and liver and kidney functional disease, presents a great challenge, but also represents a substantial unmet need.

**SUMMARY OF THE INVENTION**

The present application screens for a *Christensenella sp.* capable of effectively treating or preventing metabolic diseases by genetically predicting the integrity of the butyric acid pathway of the strain to predict its ability to produce short-chain fatty acids.

In a first aspect, the present application provides an isolated *Christensenella sp.,* which has an average nucleotide identity (ANI) value of at least 95% with the strain with a deposit number of GDMCC NO: 62509 or the strain with a deposit number of GDMCC NO: 61118, and/or has a 16S rDNA sequence with at least 98.65%, at least 98.7%, at least 98.8%, at least 98.9%, at least 99.0%, at least 99.1%, at least 99.2%, at least 99.3%, at least 99.4%, at least 99.5%, at least 99.6%, at least 99.7%, at least 99.8%, at least 99.9% or 100% identity to the sequence shown in SEQ ID NO. 1 or SEQ ID NO. 2.

In some embodiments, the *Christensenella sp.* is a novel species of the *Christensenella,* wherein a strain within the novel species has an average nucleotide identity (ANI) value of at least 95% with the strain with a deposit number of GDMCC NO: 62509 or the strain with a deposit number of GDMCC NO: 61118, for example, 95.1%, 95.2%, 95.3%, 95.4%, 95.5%, 95.6%, 95.7%, 95.8%, 95.9%, 96%, 96.1%, 96.2%, 96.3%, 96.4%, 96.5%, 96.6%, 96.7%, 96.8%, 96.9%, 97%, 97.1%, 97.2%, 97.3%, 97.4%, 97.5%, 97.6%, 97.7%, 97.8%, 97.9%, 98%, 98.1%, 98.2%, 98.3%, 98.4%, 98.5%, 98.6%, 98.7%, 98.8%, 99%, 99.1%, 99.2%, 99.3%, 99.4%, 99.5%, 99.6%, 99.7%, 99.8%, 99.9%, or 100%; and/or by 16S rDNA alignment, the 16S rDNA sequence of the strain in the species has at least 98%, at least 98.1%, at least 98.2%, at least 98.3%, at least 98.4%, at least 98.5%, at least 98.6%, at least 98.65%, at least 98.7%, at least 98.8%, at least 98.9%, at least 99.0%, at least 99.1%, at least 99.2%, at least 99.3%, at least 99.4%, at least 99.5%, at least 99.6%, at least 99.7%, at least 99.8%, at least 99.9% or 100% identity to the sequence shown in SEQ ID NO: 1 or 2.

In some embodiments, the metabolite of the *Christensenella sp.* includes a short chain fatty acid, and the short chain fatty acid comprises at least one of acetic acid, propionic acid, butyric acid, valbutyric acid, valeric acid, and hexanoic acid; preferably, the *Christensenella sp.* is capable of high-yield production of butyric acid and acetic acid; wherein the yield of butyric acid is not less than $50\mu g/g$ and/or the yield of acetic acid is not less than $300\mu g/g$; more preferably, the yield of butyric acid yield is not less than $400\mu g/g$ and/or the yield of acetic acid is not less than $2600\mu g/g$.

In some embodiments, the *Christensenella sp.* is the *Christensenella sp.* with a deposit number of GDMCC NO: 62509 or GDMCC NO: 61118.

In a second aspect, the present application provides a composition comprising the *Christensenella sp.* according to the first aspect, a culture thereof or a metabolite thereof.

In some embodiments, the culture of *Christensenella sp.* includes a solid culture, a fermentation culture, or a fermentation culture supernatant of *Christensenella sp.*

In some embodiments, the fermentation culture or fermentation culture supernatant is a fermentation culture or fermentation culture supernatant obtained by culturing in liquid medium under anaerobic conditions.

In some embodiments, the composition is provided in a liquid form or solid form.

In some embodiments, the composition comprises $1\times10^4$ cfu/mL to $1\times10^{13}$ cfu/mL or $1\times10^4$ cfu/g to $1\times10^{13}$ cfu/g of viable bacteria of *Christensenella sp.*

In some embodiments, the composition comprises $1\times10^4$ cfu/mL to $1\times10^{12}$ cfu/mL or $1\times10^4$ cfu/g to $1\times10^{12}$ cfu/g of viable bacteria of *Christensenella* sp.

In some embodiments, the composition comprises $1\times10^5$ cfu/mL to $1\times10^{11}$ cfu/mL or $1\times10^5$ cfu/g to $1\times10^{11}$ cfu/g of viable bacteria of *Christensenella* sp.

In some embodiments, the composition comprises $1\times10^6$ cfu/mL to $1\times10^{10}$ cfu/mL or $1\times10^6$ cfu/g to $1\times10^{10}$ cfu/g of viable bacteria of *Christensenella* sp.

In some embodiments, the composition comprises $1\times10^7$ cfu/mL to $1\times10^9$ cfu/mL or $1\times10^7$ cfu/g to $1\times10^9$ cfu/g of viable bacteria of *Christensenella* sp.

In some embodiments, each gram of the composition comprises $1\times10^3$ to $1\times10^{14}$ colony forming units (CFU) of bacteria; for example, comprises $1\times10^4$ to $1\times10^{12}$, $1\times10^5$ to $1\times10^{11}$, or $1\times10^6$ to $1\times10^{10}$ colony forming units (CFU) of bacteria, specifically, for example, $1\times10^3$, $2\times10^3$, $3\times10^3$, $4\times10^3$, $5\times10^3$, $6\times10^3$, $7\times10^3$, $8\times10^3$, $9\times10^3$, $1\times10^4$, $2\times10^4$, $3\times10^4$, $4\times10^4$, $5\times10^4$, $6\times10^4$, $7\times10^4$ $8\times10^4$, $9\times10^4$, $1\times10^5$, $2\times10^5$, $3\times10^5$, $4\times10^5$, $5\times10^5$, $6\times10^5$, $7\times10^5$, $8\times10^5$, $9\times10^5$, $1\times10^6$, $2\times10^6$, $3\times10^6$, $4\times10^6$, $5\times10^6$, $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, $9\times10^{13}$ or any value therebetween of the colony forming unit (CFU) of the bacteria.

In some embodiments, the *Christensenella* sp. in the composition is an attenuated bacterium, killed bacterium, lyophilized bacterium, or irradiated bacterium.

In some embodiments, the composition is in the form of a liquid, foam, cream, spray, powder (e.g., lyophilized powder), or gel.

In some embodiments, the composition is in the form of a powder, microencapsulated powder, capsule, tablet, troche, granule, oral liquid, suspension, emulsion, liquid formulation, sustained-release formulation, nanoformulation, or microencapsulated capsule.

In some embodiments, the composition is in the form of an oral agent or injectable agent.

In some embodiments, the composition further comprises one or more pharmaceutically acceptable carriers or excipients or auxiliaries.

The pharmaceutically acceptable auxiliaries are well known to those skilled in the art.

In some embodiments, the auxiliary may be at least one selected from a carrier, an excipient, a diluent, a lubricant, a wetting agent, an emulsifier, a suspension stabilizer, a preservative, a sweetener, and a flavor.

In some embodiments, the composition comprises one or more of the following: a buffering agent (e.g., sodium bicarbonate, infant formula or sterile human milk or other reagents that allow for bacterial survival and growth (for example, survival in the acidic environment of the stomach and growth in the intestinal environment)), a lyoprotectant, a preservative, a stabilizing agent, a binder, a compacting agent, a lubricant, a dispersion enhancer, a disintegrating agent, an antioxidant, a flavoring agent, a sweetener, and a coloring agent.

In some embodiments, the composition further comprises one or more additional active agents for the prevention or treatment of metabolic diseases.

The additional active agent has at least one of the following functions: (a) appetite suppression, (b) prevention of metabolic diseases, and (c) treatment of metabolic diseases.

In some embodiments, the appetite suppression comprises reducing the amount of food intake and/or reducing appetite.

In some embodiments, the additional active agent is selected from: a GLP-1 receptor agonist, a GLP-1 receptor and GCG receptor dual agonist, a GLP-1 receptor, GIP receptor, and GCG receptor triple agonist, an AMPK agonist, or an active drug promoting GLP-1 secretion.

In some embodiments, the additional active agent is selected from: metformin, sulfonylureas, meglitinides, thiazolidinediones, DPP-4 inhibitor, GLP-1 receptor agonist, SGLT2 inhibitor, insulin, pioglitazone, rosiglitazone, pentoxifylline, $\Omega$-3 fatty acid, statins, ezetimibe, or ursodeoxycholic acid, semaglutide, liraglutide, exenatide, and benaglutide.

In some embodiments, the additional active agent is metformin.

In some embodiments, the composition may be formulated as a frozen composition, such as a frozen composition made by flash freezing and drying, or lyophilization, for storage and/or transportation.

In some embodiments, the composition is obtained by spray drying. In some embodiments, the composition is obtained by electrostatic spray drying.

In some embodiments, the strain in the composition is freeze-dried or spray-dried. In some embodiments, the strain in the composition is electrostatic spray-dried. In some embodiments, the strain in the composition is freeze-dried or spray-dried and remains viable. In some embodiments, the strain in the composition is freeze-dried or spray-dried and is capable of partially or fully colonizing in the intestine. In some embodiments, the strain is subjected to reconstitution prior to administration. In some embodiments, the reconstitution is performed using a diluent as described herein.

In some embodiments, the composition may be administered alone or in combination with a carrier such as a pharmaceutically acceptable carrier or a biocompatible scaffold.

In some embodiments, the composition is formulated for oral administration. In some embodiments, the composition is an enteric formulation. In some embodiments, the enteric formulation is a dosage form with an enteric coating. For example, the enteric formulation may be an enteric-coated granule, enteric-coated tablet or enteric-coated capsule. In some embodiments, the composition is a capsule formulation. In some embodiments, the capsule formulation is a hard capsule or a soft capsule; or the capsule formulation is a sustained-release capsule, a controlled-release capsule, or an enteric-coated capsule, or alternatively, the capsule formulation may be a microencapsulated capsule, or a microcapsule.

In some embodiments, the composition is a medicament, a health care product or a food product.

In some embodiments, the composition is in an infant-applicable dosage form, a child-applicable dosage form, or an adult-applicable dosage form.

In some embodiments, the composition is in a dosage form of gastrointestinal administration or a dosage form of parenteral administration.

In a third aspect, the present application provides use of the *Christensenella sp.* according to the first aspect or the composition according to the second aspect in the preparation of a medicament, health care product, or food product for treating, preventing, or alleviating metabolic disease or disease caused by metabolic disorder.

In some embodiments, the metabolic disease, metabolic disorder, or disease caused by metabolic disorder includes, but is not limited to, at least one of the following: liver disease, obesity and obesity-related disease, cardiovascular disease, diabetes, dyslipidemia, cardiovascular and cerebrovascular diseases, glucose intolerance, atherosclerosis, coronary heart disease, or hypertension, type I diabetes, type II diabetes, abnormal glucose tolerance, insulin resistance, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic diseases, dyslipidemia, non-alcoholic fatty liver disease (NAFLD), non-alcoholic steatohepatitis (NASH), atherosclerosis, or nephropathy.

In some embodiments, the liver disease includes, but is not limited to, at least one of the following: fatty liver, NAFLD/NASH, abnormal liver function, extrahepatic cholestasis, hepatitis, liver injury, intrahepatic cholestasis, liver fibrosis, cirrhosis, and liver cancer.

In some embodiments, the *Christensenella sp.* of the present application may be used for the treatment or prevention of liver function impairment-related diseases, including at least one of the following diseases: fatty liver, non-alcoholic fatty liver disease, non-alcoholic steatohepatitis, liver fibrosis, cirrhosis, and liver cancer.

In some embodiments, a trigger of the liver disease includes, but is not limited to, at least one of the following: high-fat diet, high-cholesterol diet, high-sugar diet, hyperlipidemia, hyperglycemia, or high cholesterol.

In some embodiments, the liver disease includes high-fat diet-induced, high cholesterol diet-induced, high-sugar diet-induced, combination of high-fat and high-cholesterol-induced, combination of high-fat and high-sugar-induced, and/or combination of high-fat, high cholesterol and high sugar-induced.

In some embodiments, the *Christensenella sp.* of the present application may be used for the treatment, prevention or alleviation of liver function impairment-related diseases, including at least one of the following diseases: fatty liver, non-alcoholic fatty liver disease, non-alcoholic steatohepatitis, liver fibrosis, cirrhosis, and liver cancer.

In some embodiments, the obesity and obesity-related diseases include, but are not limited to: overweight, obesity, metabolic syndrome, cardiovascular disease, cardiovascular and cerebrovascular diseases, hyperlipidemia, hypercholesterolemia, hypertension, insulin resistance syndrome, obesity-associated gastroesophageal reflux disease, and steatohepatitis.

In some embodiments, a trigger of obesity and obesity-related diseases includes, but is not limited to, at least one of the following: high-fat diet, high-sugar diet, high cholesterol, hyperlipidemia, hyperglycemia, NAFLD, or NASH.

In some embodiments, the obesity and obesity-related diseases include, but are not limited to: obesity induced by high-fat diet, obesity induced by high cholesterol, obesity induced by high-sugar diet, obesity induced by combination of high-fat and high cholesterol, obesity induced by combination of high-fat and high sugar, obesity induced by combination of high-fat, high cholesterol and high sugar, or obesity in a patient with NAFLD or NASH.

In some embodiments, the obesity and obesity-related diseases include at least one of the following: obesity, metabolic syndrome, cardiovascular disease, hyperlipidemia, hypercholesterolemia, hypertension, insulin resistance syndrome, obesity-associated gastroesophageal reflux disease, and steatohepatitis.

In some embodiments, the obesity and obesity-related diseases include at least one of the following: obesity, metabolic syndrome, cardiovascular disease, hyperlipidemia, hypercholesterolemia, hypertension, insulin resistance syndrome, obesity-associated gastroesophageal reflux disease, and steatohepatitis.

In some embodiments, the cardiovascular disease or cardiovascular and cerebrovascular disease includes, but is not limited to: atherosclerosis, coronary heart disease, cardiovascular disease in NAFLD or NASH patient, cardiovascular and cerebrovascular disease in a patient with NAFLD or NASH, and high cholesterol disease.

In some embodiments, a trigger of cardiovascular disease or cardiovascular and cerebrovascular disease includes, but is not limited to, at least one of the following: atherosclerosis, NAFLD, NASH, hyperlipidemia, hyperglycemia, or high cholesterol.

In some embodiments, the diabetes includes, but is not limited to: type I diabetes, type II diabetes, gestational diabetes, HDAC activity-mediated diabetes, diabetic nephropathy, diabetic neuropathy, diabetic ophthalmopathy, diabetic retinopathy, diabetic foot, diabetes induced by damage to pancreatic β-cells, diabetes induced by insulin resistance, and diabetes induced by obesity.

In some embodiments, a trigger of diabetes includes, but is not limited to, at least one of the following: pancreatic islet cell dysfunction, decreased insulin secretion, increased insulin resistance, high-fat diet, high-sugar diet, high cholesterol, hyperlipidemia, hyperglycemia, NAFLD, or NASH.

In some embodiments, a trigger of diabetes includes at least one of the following: high-fat diet induced, high-sugar diet induced, or high-cholesterol diet induced.

In some embodiments, the diabetes is characterized by hypoglycemia resulting from low levels of insulin and/or peripheral insulin resistance.

In some embodiments, the metabolic disorder includes, but is not limited to: (1) diabetes induced by disorder of glucose metabolism, or (2) diabetes induced by abnormal glucose tolerance or reduced glucose tolerance, or (3) diabetes induced by damage to pancreatic β-cells, or (4) diabetes induced by insulin resistance.

In some embodiments, the *Christensenella sp.* of the present application has the effect of increasing the secretion level of glucagon-like peptide-1 (GLP-1), thereby regulating the body's glucose homeostasis, improving the body's glucose tolerance, further improving the body's insulin sensitivity and leptin sensitivity, and thereby achieving the prevention and/or treatment of diabetes and/or hyperlipidemia.

In some embodiments, the medicament or health care product or food product has at least one effect selected from the following:

    reducing liver weight;
    treating initial steatohepatitis lesions;
    slowing down the accumulation of fat in liver cells;
    decreasing serum AST and ALT levels;
    reducing inflammatory lesions in abdominal white adipose tissue;
    decreasing body weight in mammals;
    reducing food intake in mammals;
    lowering body fat in mammals;
    lowering the levels of at least one indicator selected from the following in mammalian serum: total cholesterol, low-density lipoprotein and triglycerides;
    increasing the level of high-density lipoprotein in mammalian serum;
    improving impaired oral glucose tolerance in mammals;
    lowering fasting blood glucose level in mammals;
    lowering HOMA-IR index in mammals;
    repairing gastrointestinal mucosal damage;
    treating, preventing, or alleviating coronary heart disease;

treating, preventing, or alleviating atherosclerosis
treating, preventing, or alleviating hyperglycemia;
treating, preventing, or alleviating hyperlipidemia;
treating, preventing, or alleviating high cholesterol;
treating, preventing, or alleviating liver function impairment;
treating, preventing, or alleviating fatty liver;
treating, preventing, or alleviating NAFLD or NASH;
treating, preventing, or alleviating hypertension;
treating, preventing or alleviating diabetes, preferably gestational diabetes or type II diabetes or HDAC activity-mediated diabetes;
treating, preventing, or alleviating obesity;
treating, preventing, or alleviating metabolic syndrome;
treating, preventing, or alleviating localized sebum excess, inguinal fat excess, epididymal fat excess, and/or brown adipose excess.

In a fourth aspect, the present application provides use of *Christensenella sp.,* a culture thereof, or a metabolite thereof in the preparation of GLP-1 receptor agonist, wherein the *Christensenella sp.* has one or more characteristics selected from the following:

a 16S rDNA sequence with at least 99% identity to the sequence shown in SEQ ID NO.3; and
the *Christensenella sp.* is Gram-negative.

Preferably, the *Christensenella sp.* comprises the strain with a deposit number of GDMCC NO: 61117.
Preferably, the culture of the *Christensenella sp.* is a culture supernatant of *Christensenella sp.* or a fermentation supernatant of *Christensenella sp.*
Preferably, the *Christensenella sp.* is *Christensenella intestinihominis.*
In a fifth aspect, the present application provides use of *Christensenella sp.,* a culture thereof, or a metabolite thereof in the preparation of DPP4 (DPPIV) inhibitor, wherein the *Christensenella sp.* has a 16S rDNA sequence with at least 99% identity to the sequence shown in SEQ ID NO. 3; and the *Christensenella sp.* is Gram-negative.
Preferably, the *Christensenella sp.* comprises the strain with a deposit number of GDMCC NO: 61117.
Preferably, extract of the *Christensenella sp.* is a culture supernatant of *Christensenella sp.* or a fermentation supernatant of *Christensenella sp.* or a secondary metabolite of *Christensenella sp.*
Preferably, the *Christensenella sp.* is optionally *Christensenella intestinihominis.*
In a sixth aspect, the present application provides use of the *Christensenella sp.,* the composition, the GLP-1 receptor agonist, or the DPP4 (DPPIV) inhibitors in the preparation of a composition for increasing energy expenditure in a subject by modulating adipose tissue metabolism and/or glucose homeostasis.
Preferably, the *Christensenella sp.,* the composition, the GLP-1 receptor agonist, or the DPP4 (DPPIV) inhibitor does not affect the food intake, more preferably, does not affect food intake amount, in the subject.
Preferably, the composition is a nutritional composition.
Preferably, the *Christensenella sp.,* the composition, the GLP-1 receptor agonist or the DPP4 (DPPIV) inhibitor is in the form of food additive, dietary supplement, nutritional product or medical food.
In a seventh aspect, the present application provides use of the *Christensenella sp.,* the composition, the GLP-1 receptor agonist, or the DPP4 (DPPIV) inhibitor in the preparation of a composition for promoting weight loss in a subject.
Preferably, the *Christensenella sp.,* the composition, the GLP-1 receptor agonist, or the DPP4 (DPPIV) inhibitor does not affect the food intake of the subject, and more preferably, does not affect the food intake amount of the subject.
Preferably, the composition is a nutritional composition.
Preferably, the *Christensenella sp.,* the composition, the GLP-1 receptor agonist or the DPP4 (DPPIV) inhibitor is in the form of food additive, dietary supplement, nutritional product or medical food.
In an eighth aspect, the present application provides use of the *Christensenella sp.,* the composition, the GLP-1 receptor agonist, or the DPP4 (DPPIV) inhibitor in promoting weight loss in a subject; wherein the *Christensenella sp.* is Gram-negative.
Preferably, the *Christensenella sp.,* the composition, the GLP-1 receptor agonist, or the DPP4 (DPPIV) inhibitor does not affect the food intake of the subject, and more preferably, does not affect the food intake amount of the subject.
Preferably, the composition is a nutritional composition.
Preferably, the *Christensenella sp.,* the composition, the GLP-1 receptor agonist or the DPP4 (DPPIV) inhibitor is in the form of food additive, dietary supplement, nutritional product or medical food.
In some embodiments, the *Christensenella sp.,* the composition, the GLP-1 receptor agonist, or the DPP4 (DPPIV) inhibitor is administered in combination with one or more additional probiotic and/or one or more prebiotics.

In a ninth aspect, the present application provides a *Christensenella sp.,* or a composition comprising *Christensenella sp.,* a culture thereof, or a metabolite thereof for use in increasing energy expenditure in a subject, wherein the *Christensenella sp.* is selected from the *Christensenella sp.* according to the first aspect, and the composition is selected from the composition according to the second aspect.

Preferably, the *Christensenella sp.* or the composition does not affect the food intake of the subject, and more preferably, the *Christensenella sp.* or the composition does not affect the food intake amount of the subject.

Preferably, the composition is a nutritional composition.

Preferably, the *Christensenella sp.* or the composition is in the form of food additive, dietary supplement, nutritional product or medical food.

## BRIEF DESCRIPTION OF THE DRAWINGS

The following will describe the examples in combination with the drawings, so as to make the above and other aspects and advantages of the present invention more apparent and easier to understand.

Figure 1: A shows the Gram staining of strain MNH05119, and B shows the electron micrograph of strain MNH05119.

Figure 2: A shows the Gram staining of strain MNH06163, and B shows the electron micrograph of strain MNH06163.

Figure 3: positions of strains MNH05119, MNH06163, and MNH04863 in the phylogenetic tree.

Figure 4: showing the pH tolerance (Figure 4A), NaCl tolerance (Figure 4B), and bile salt tolerance (Figure 4C) of strains MNH05119, MNH06163, and MNH04863, respectively.

Figure 5: A and B show that strains MNH05119 and MNH06163 reduce serum ALT levels, respectively, and C shows that strain MNH05119 reduces serum AST levels. Data are presented as mean $\pm$ standard deviation (Mean $\pm$ SD). Statistical analysis was performed using the student's t-test; and *$p < 0.05$ compared to HFD-control group.

Figure 6: showing that strains MNH05119 and MNH06163 can reduce liver weight in obesity mice.

Figure 7: showing effects of strains MNH05119, MNH06163, and MNH04863 on oral glucose tolerance in high-fat diet-induced obesity mice. A: oral glucose tolerance results; and B: area under the curve for oral glucose tolerance. Data are presented as mean $\pm$ SD. Statistical analysis was performed using the student's t-test; *$p < 0.05$ and ****$p < 0.0001$, compared to HFD-control group.

Figure 8: showing effects of MNH05119 and MNH06163 on fasting blood glucose in high-fat diet-induced obesity mice. A: MNH05119 significantly reduces fasting blood glucose in high-fat diet-induced obesity mice; and B: MNH06163 significantly reduces fasting blood glucose in high-fat diet-induced obesity mice. Data are presented as mean $\pm$ standard deviation (Mean $\pm$ SD). Statistical analysis was performed using the student's t-test; and *$p < 0.05$ compared to HFD-control group.

Figure 9: effects of MNH05119 and MNH06163 on insulin resistance in high-fat diet-induced obesity model mice. A: HOMA-IR index; B: Resistin; and C: glucose-dependent insulinotropic polypeptide (GIP). Data are presented as mean $\pm$ standard deviation (Mean $\pm$ SD). Statistical analysis in A was performed using student's t-test; one-way ANOVA with Dunnett's multiple comparison test was used for B and C. No significance (ns) is not shown; and significance of differences is indicated by *, *$p < 0.05$, **$p < 0.01$, ***$p < 0.001$, and ****$p < 0.0001$.

Figure 10: effects of MNH05119 and MNH04863 on serum LPS levels in high-fat diet-induced obesity mice. Data are presented as mean $\pm$ standard deviation (Mean $\pm$ SD). One-way ANOVA with Dunnett's multiple comparison test was used. No significance is not shown; and significance of differences is indicated by *, *$p < 0.05$, **$p < 0.01$, ***$p < 0.001$, and ****$p < 0.0001$.

Figure 11: effects of MNH05119 and MNH04863 on serum leptin levels in high-fat diet-induced obesity mice. *$p < 0.05$, **$p < 0.01$, ***$p < 0.001$, and ****$p < 0.0001$, compared to HFD-control group.

Figure 12: MNH04863 significantly increases plasma GLP-1 concentration in high-fat diet-induced obesity mice.

Figure 13: MNH04863 significantly downregulates the relative ratio of DPP4 (DPPIV)/CD26 in serum.

Figure 14: effects of combination of MNH05119 with Metformin on reduceing oral glucose tolerance in type II diabetic mice. A: oral glucose tolerance results; B: area under the curve for oral glucose tolerance. Data are presented as mean $\pm$ standard deviation (Mean $\pm$ SD). Statistical analysis was performed using one-way ANOVA with Dunnett's multiple comparison test. *$p < 0.05$, and ***$p < 0.001$.

Figure 15: effects of combination of MNH05119 with Metformin on fasting blood glucose levels in type II diabetic mice. Data are presented as mean $\pm$ standard deviation (Mean $\pm$ SD). Statistical analysis was performed using one-way ANOVA method with Dunnett's multiple comparison test. *$p < 0.05$, and ****$p < 0.0001$.

Figure 16: effects of MNH05119 and MNH06163 on body weight in high-fat diet-induced obesity mice. A: body weight results; and B: percentage change in body weight. Data are presented as mean $\pm$ standard deviation (Mean $\pm$ SD). Statistical analysis was performed using the student's t-test; and *$p < 0.05$ compared to HFD-control group.

Figure 17: effects of MNH05119 and MNH06163 on food intake amount in high-fat diet-induced obesity model mice.

Figure 18: effects of MNH05119 and MNH06163 on concentrations of triglyceride (TG), high-density lipoprotein cholesterol, and low-density lipoprotein cholesterol in high-fat diet-induced obesity model mice. A: results of serum triglycerides (TG); B: results of serum total cholesterol; C: results of serum high-density lipoprotein cholesterol (HDL-C); and D: results of serum low-density lipoprotein cholesterol (LDL-C). Data are presented as mean $\pm$ standard deviation (Mean $\pm$ SD). Statistical analysis was performed using the student's t-test; and *p*< 0.05 compared to HFD-control group.

Figure 19: effects of MNH05119 and MNH06163 on fat weight in high-fat diet-induced obesity model mice. A: subcutaneous fat results; B: inguinal fat results; C: epididymal fat results; and D: scapular fat results. Data are presented as mean $\pm$ standard deviation (Mean $\pm$ SD). Statistical analysis was performed using the student's t-test; and *p*< 0.05 compared to HFD-control group.

Figure 20: 10% crude extract CFS of MNH04863 significantly induces GLP-1 expression.

## Deposition of strains

Strain MNH05119 (also known as MNO-119, MNH-119) was deposited at Guangdong Microbial Culture Collection Center (GDMCC) with a deposit number of GDMCC NO: 62509, conserved on June 1, 2022, the address is Guangdong Institute of Microbiology, 5th Floor, Building 59th, 100, Xianlie Middle Road, Guangzhou, and taxonomic designation is *Christensenella sp.*

Strain MNH06163 (also known as MNO-163, MNH-163) was deposited at Guangdong Microbial Culture Collection Center (GDMCC) with a deposit number of GDMCC NO: 61118, conserved on August 4, 2020, the address is Guangdong Institute of Microbiology, 5th Floor, Building 59th, 100, Xianlie Middle Road, Guangzhou, and taxonomic designation is *Christensenella sp.*

Strain MNH04863 (also known as MNO-863, MNH-863) was deposited at Guangdong Microbial Culture Collection Center (GDMCC) with a deposit number of GDMCC NO: 61117, conserved on August 4, 2020, the address is Guangdong Institute of Microbiology, 5th Floor, Building 59th, 100, Xianlie Middle Road, Guangzhou, and taxonomic designation is *Christensenella sp.*

## DETAILED DESCRIPTION OF THE EMBODIMENTS

The present application has isolated two novel strains of *Christensenella,* namely, the strain with a deposit number of GDMCC NO: 62509 and the strain with a deposit number of GDMCC NO: 61118, and has conducted their identification using traditional taxonomic and molecular biological methods. The identification results indicate that these two strains belong to a novel species within the *Christensenella.* Furthermore, the present application has investigated the biochemical properties and therapeutic uses of these two strains.

It is known in the art that taxonomic identification of species can be performed using traditional taxonomic methods and molecular biological methods. Traditional taxonomic methods include, but are not limited to, such as cell morphology observation, Gram staining, flagella staining, and various metabolic assays. Molecular biological methods include, but are not limited to, ribosomal RNA sequencing, whole genome sequencing-based methods and the like.

16S rRNA is a ribosomal RNA in prokaryotes, and the 16S rRNA gene consists of a variable region and a conserved region, the conserved region is shared by all bacteria, while the variable region varies to different degrees among different bacteria. By alignment of the 16S rRNA gene sequences of bacteria, a phylogenetic tree can be constructed according to their evolutionary distance based on the base of sequence differences. When the identity between the 16S rRNA gene sequences of two strains is less than 98.65%, they can be judged to belong to different species (see, Kim, M., Oh, H.-S., Park, S.-C., & Chun, J. (2014). Towards a taxonomic coherence between average nucleotide identity and 16S rRNA gene sequence similarity for species demarcation of prokaryotes. International Journal of Systematic and Evolutionary Microbiology, 64(Pt 2), 346-351, and Liu, C., Du, M.-X., Abuduaini, R., Yu, H.-Y., Li, D.-H., Wang, Y.-J., Liu, S.-J. (2021). Enlightening the taxonomy darkness of human gut microbiomes with a cultured biobank. Microbiome, 9(1), p.23).

The "identity" between the sequences of two nucleic acid molecules can be determined using known computer algorithms, such as the "FASTA" program, the GCG package, BLASTN or FASTA. Commercially or publicly available programs may also be, for example, the DNAStar "MegAlign" program.

Next-generation sequencing technology can also be used for bacterial species identification based on whole-genome sequencing, making the identification results of species more accurate. Average nucleotide identity (ANI) of bacterial genomes refers to the similarity of homologous genes between two bacterial genomes. ANI values can be calculated by methods such as BLAST. In the field of bacterial taxonomy, it is widely accepted that an ANI value of above 95% is required to identify as belonging to the same species (Jain C, Rodriguez-R L M, Phillippy A M, et al. High throughput ANI analysis of 90K prokaryotic genomes reveals clear speciesboundaries[J]. Nature Communications, 2018, 9(1): 5114.)

Various established tools for calculating ANI values are available currently, such as local computational software Jspecies (/jspecies) and Gegenees (/documentation.html), and the online calculator ANI caculator (http:/enveomics.gatech.edu/), EzGenome (/ezgenome/ani) and ANItools.

The present application relates to a culture of bacterial strain. The term "culture" refers to the product obtained by culturing an isolated strain in a medium. The medium may be selected from natural or synthetic medium, for example it may be a solid medium or a liquid medium. In some embodiments, the medium comprises peptone, such as at least one of meat peptone, casein peptone, whey peptone, soy peptone and the like, and additionally, it may include other inorganic salts or organic substances that promote bacterial growth.

The culture may be a medium after the strain has been cultured, or a supernatant obtained from the medium through centrifugation, or a concentrated product obtained after the medium or the supernatant thereof has been subjected to at least one of evaporation, lyophilization, dialysis, extraction, membrane separation, etc., or an extract obtained after the medium or the supernatant thereof has been subjected to at least one of extraction, solvent extraction (e.g., water or organic solvents), or a dried product obtained after any one of the medium, supernatant thereof, concentrated product, or extract has been subjected to dry. It should be understood that the extract may be an extraction directed to a specific one or more of the components thereof, such as an extraction directed to a component of a specific molecular weight range, a specific solubility, a specific isoelectric point, etc.; or it may be an extraction not directed to a particular type or types of components. The specific extraction method includes the extraction using at least one of the solvents such as methanol, ethanol, chloroform, water, acid, alkali, etc. according to a specific process protocol under certain temperature conditions, such as extraction in 75% v/v ethanol at 60°C~80°C for 1~10min, or extraction in methanol at -30°C~-10°C for 1~10min, or extraction in a methanol/chloroform/water solution at -40°C~-20°C for 30~60 min, or extraction in deionized water at 95°C for 1~10 min, or extraction in perchloric acid, trichloroacetic acid, hydrochloric acid, or sodium hydroxide solution at 0~4°C, etc., so as to extract therefrom the corresponding metabolite, such as a short-chain fatty acid or a short-chain fatty acid salt, e.g., at least one of acetic acid, butyric acid, valeric acid, acetate, propionate, or valerate.

Using the methods described above, those skilled in the art can determine whether an isolated strain belongs to a novel species of *Christensenella* identified by the present inventors. For example, when the average nucleotide identity ANI value to the strain with a deposit number of GDMCC NO: 62509 or the strain with a deposit number of GDMCC NO: 61118 is at least 95%, for example, 95.1%, 95.2%, 95.3%, 95.4%, 95.5%, 95.6%, 95.7%, 95.8%, 95.9%, 96%, 96.1%, 96.2%, 96.3%, 96.4%, 96.5%, 96.6%, 96.7%, 96.8%, 96.9%, 97%, 97.1%, 97.2%, 97.3%, 97.4%, 97.5%, 97.6%, 97.7%, 97.8%, 97.9%, 98%, 98.1%, 98.2%, 98.3%, 98.4%, 98.5%, 98.6%, 98.7%, 98.8%, 99%, 99.1%, 99.2%, 99.3%, 99.4%, 99.5%, 99.6%, 99.7%, 99.8%, 99.9%, or 100%, it can be determined to belong to the same species. For example, when the average nucleotide identity ANI value to the strain with a deposit number of GDMCC NO: 61117 is at least 95%, for example, 95.1%, 95.2%, 95.3%, 95.4%, 95.5%, 95.6%, 95.7%, 95.8%, 95.9%, 96%, 96.1%, 96.2%, 96.3%, 96.4%, 96.5%, 96.6%, 96.7%, 96.8%, 96.9%, 97%, 97.1%, 97.2%, 97.3%, 97.4%, 97.5%, 97.6%, 97.7%, 97.8%, 97.9%, 98%, 98.1%, 98.2%, 98.3%, 98.4%, 98.5%, 98.6%, 98.7%, 98.8%, 99%, 99.1%, 99.2%, 99.3%, 99.4%, 99.5%, 99.6%, 99.7%, 99.8%, 99.9%, or 100%, it can be determined to belong to the same species.

For example, when the 16S rDNA sequence thereof has at least 98.65% identity to the sequence shown in SEQ ID NO. 1 or 2, such as at least 98.7%, 98.8%, 99%, 99.1%, 99.2%, 99.3%, 99.4%, 99.5%, 99.6%, 99.7%, 99.8%, 99.9%, or 100%, it can be determined to belong to the same species.

"Strain" refers to a member of a bacterial species with genetic characteristics that make it distinguishable from closely related members of the same bacterial species. Genetic characteristics can be the complete or partial absence of at least one gene, the complete or partial absence of at least one regulatory region (e.g., promoter, terminator, riboswitch, ribosome-binding site), the absence of at least one natural plasmid ("cure"), the presence of at least one recombinant gene, the presence of at least one mutated gene, the presence of at least one exogenous gene (a gene from another species), the presence of at least one mutated regulatory region (e.g., promoter, terminator, riboswitch, ribosome-binding site), the presence of at least one unnatural plasmid, the presence of at least one antibiotic resistance cassette, or any combination thereof. Genetic characteristics between different strains can be identified by PCR amplification, optionally followed by DNA sequencing of the genomic region of interest or the whole genome. In cases where a strain (compared to another strain of the same species) gains or loses antibiotic resistance or gains or loses biosynthetic capacity (e.g., nutrient-deficient strains), the strain or nutrient/metabolite can be distinguished by selection or counter-selection using antibiotics.

"Supernatant liquid" or "supernatant" as used herein refers to a culture supernatant of a bacterial strain according to the present application, optionally comprising compounds and/or cellular debris of the strain, and/or metabolites and/or molecules secreted by the strain.

Composition can be prepared using the *Christensenella sp.* of the present application, e.g., prepared by using a pharmaceutically acceptable auxiliary. The pharmaceutical composition comprises a pharmaceutically effective amount of the *Christensenella sp.*, for example, *Christensenella sp.* with a deposit number of GDMCC NO: 62509 or *Christensenella sp.* with a deposit number of GDMCC NO: 61118. Similarly, the *Christensenella sp.* with a deposit number of GDMCC NO: 61117 can also be prepared as a pharmaceutical composition, for example, prepared by using a pharmaceutically acceptable auxiliary, comprising a pharmaceutically effective amount of the *Christensenella sp.*

A suitable pharmaceutically acceptable auxiliary that may be used include, for example, carrier, excipient, diluent, lubricant, wetting agent, emulsifier, suspension stabilizer, preservative, sweetener, and flavor.

The composition of the present application may be formulated in any form suitable for enhancing the abundance of *Christensenella sp.* in a subject. The composition may be administered via various routes including oral administration (e.g., by oral gavage), intramuscular injection, inhalation, intracranial, intralymphatic, intraocular, intraperitoneal, intrapleural, intrathecal, intratracheal, intrauterine, intravascular, intravenous, intravesical, intranasal, gastrointestinal, biliary perfusion, cardiac perfusion, pre-anal, rectal, spinal subcutaneous, sublingual, topical, intravaginal, transdermal, ureteral, or urethral and the like.

Examples of suitable dosage forms for the composition of the present application include, but are not limited to, tablets, aerosols, chewable sticks, capsules, capsules containing coated particles, capsules containing sustained-release particles, capsules containing sustained-release particles, tablets, chewable tablets, tablets containing coated particles, dispersible tablets, effervescent tablets, sustained-release tablets, orally disintegrating tablets, tampons, tapes, or cannulas/troches.

In some embodiments, the composition is a sugar-coated tablet, gel capsule, gel, emulsion, tablet, tablet capsule, hydrogel, nanofiber gels, electrospun fiber, food bar, candy, fermented milk, fermented cheese, chewing gum, powder, or toothpaste and the like.

In some embodiments, the administration may also be conducted by inclusion in the subject's diet, such as inclusion in a functional food for use in humans or companion animals.

The composition of the present application may comprise a pharmaceutically acceptable excipient, diluent or carrier. The composition may also include an antioxidant and suspending agent.

In some embodiments, the *Christensenella sp.* in the composition of the present application is lyophilized. In some embodiments, the *Christensenella sp.* in the composition of the present application is spray-dried. In some embodiments, the *Christensenella sp.* in the composition of the present application is lyophilized or spray-dried and is viable. In some embodiments, the *Christensenella sp.* in the composition of the present application is lyophilized or spray-dried and are capable of partially or fully colonizing in the intestine. In some embodiments, the lyophilized *Christensenella sp.* is reconstituted prior to administration. In some embodiments, the reconstitution is performed using a diluent as described herein.

In some embodiments, the composition of the present application is administered orally. Pharmaceutical dosage forms suitable for oral administration include solid boluses, solid microparticles, semi-solids and liquids (including multiphase or dispersible systems), tablets; soft or hard capsules containing multiple particles or nanoparticles, liquids (e.g., aqueous solutions), emulsions, or powders; troches (including liquid filler); chewables; gels; fast-dispersing dosage forms; sprays; and buccal/mucosal adhesion patches.

In some embodiments, the composition is an enteric formulation, i.e., gastric fluid-tolerant formulations (e.g., tolerant to gastric pH) suitable for delivery of the composition of the present application to the intestines by oral administration. The enteric formulation may be particularly useful when the *Christensenella sp.* or component of the composition is acid-sensitive, for example readily degradable under gastric conditions.

In some embodiments, the enteric formulation is a dosage form comprising an enteric coating. For example, the formulation is an enteric-coated tablet or enteric-coated capsule, etc.

In some embodiments, the composition is in the form of a soft capsule.

In some embodiments, the composition is in form of microencapsulated capsule.

The composition of the present application is selected from pharmaceutical composition, health care product or food product.

A subject of the present application may be a human or an animal, the animal including, but not limited to, a cow, a sheep, a cat, a canine, a horse, a rabbit, a monkey, a mouse, a rat, an alpaca, a camel, and the like.

The pharmaceutical composition of the present application may be used to treat, prevent, or alleviate metabolic diseases or diseases caused by metabolic disorders.

In some embodiments, the metabolic diseases, metabolic disorders, or diseases caused by metabolic disorders include, but are not limited to at least one of the following: liver diseases, obesity and obesity-related diseases, cardiovascular diseases, diabetes, dyslipidemia, cardiovascular and cerebrovascular diseases, glucose intolerance, atherosclerosis, coronary heart disease or hypertension, type I diabetes, type II diabetes, abnormal glucose tolerance, insulin resistance, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic diseases, dyslipidemia, non-alcoholic fatty liver disease (NAFLD), non alcoholic steatohepatitis (NASH), atherosclerosis, or nephropathy.

Diabetes

Diabetes includes Type I diabetes (T1D), Type II diabetes (T2D), and gestational diabetes mellitus (GDM). Type I diabetes is a diabetes caused by autoimmune damage or idiopathic causes, characterized by absolute destruction of pancreatic islet function, mostly occurring in children and adolescents, and requires insulin therapy to achieve a satisfactory outcome, or it may be life-threatening. Type II diabetes is a multifactorial syndrome characterized by abnormalities in carbohydrate/fat metabolism, which usually includes hyperglycemia, hypertension, and cholesterol abnormalities. Type II diabetes is the result of ineffective insulin action (reduced receptor binding), so it is important to test not only the fasting blood glucose, but also to observe the postprandial 2-hour blood glucose, and in particular, pancreatic function assessments should be done. There are two conditions of diabetes during pregnancy: one is diabetes diagnosed prior to pregnancy, called "diabetes in pregnancy"; the other is diabetes with normal glucose metabolism or potentially decreased glucose tolerance before pregnancy, which only appears or is diagnosed during pregnancy, also called "gestational diabetes mellitus (GDM)". More than 80% of diabetic pregnancies are classified as GDM.

Four metabolic disease-related models, high-fat diet (HFD)-induced mouse obesity model, high-fat, high-sugar, and high-cholesterol-induced mouse NASH model, high-fat diet combined with streptozotocin (HFD-STZ)-induced mouse type II diabetes model, and leptin receptor gene-deficient mouse model (db/db), are all commonly used in metabolic disease mouse model. The model mouse typically exhibit characteristics such as obesity, insulin resistance, hyperglycemia, hyperlipidemia, high cholesterol, NAFLD/NASH and other metabolic diseases.

Insulin resistance refers to a condition where, for various reasons, the efficiency of insulin in promoting glucose uptake and utilization decreases. The body compensates by secreting excessive amounts of insulin, leading to hyperinsulinemia to maintain blood glucose stability. Insulin resistance is prone to metabolic syndrome and type II diabetes.

Oral glucose tolerance is used to determine the function of pancreatic β-cells and the body's ability to regulate blood glucose, and is currently recognized as a diagnostic indicator for diabetes. When the glucose metabolism is disordered, after ingesting a certain amount of glucose orally, blood glucose either rises sharply or does not rise significantly, while can not be reduced to the fasting level or the original level within a short period. This condition is referred to as abnormal glucose tolerance or decreased glucose tolerance. Abnormal glucose tolerance indicates that the reduced glucose metabolism capacity of body, commonly found in type II diabetes and obesity.

HOMA-IR is an index used to evaluate the level of insulin resistance in an individual, and is now widely used in clinical settings to assess insulin sensitivity in diabetic patients, which is calculated as: fasting blood glucose level (FPG, mmol/L) $\times$ fasting insulin level (FINS, μU/mL)/22.5. The HOMA-IR index is 1 in a normal individual. As the level of insulin resistance increases, the HOMA-IR index will be higher than 1.

L cells in the intestinal tract are capable of secreting glucagon-like peptide-1 (GLP-1), which promotes insulin production by pancreatic β-cells and inhibits glucagon production by pancreatic α-cells, thereby regulating the body's glucose homeostasis and improving the body's glucose tolerance.

Liver Function Diseases

Liver function diseases is abnormal liver function or liver function impairment. Alanine aminotransferase (ALT) and/or aspartate aminotransferase (AST) are sensitive markers for liver function disease. Significant elevation of ALT and/or AST levels in the blood indicate in the presence of abnormal liver function (such as liver injury, non-alcoholic fatty liver disease (NAFLD), or non-alcoholic steatohepatitis (NASH)). In general, ALT reflects acute liver injury with higher sensitivity than AST. Persistent elevation of ALT suggests chronic liver injury. In cases of chronic hepatitis, cirrhosis, and liver cancer, the AST level may rise markedly and even exceed the ALT level. AST level indicate the chronicity, extensiveness and severity of liver lesions and even suggest the prognosis of chronic liver diseases.

Common liver diseases with elevated ALT and/or AST include: acute viral hepatitis (hepatitis A, hepatitis B, hepatitis C, hepatitis D, and hepatitis E); EB virus and cytomegalovirus infections; chronic hepatitis B or chronic hepatitis C; autoimmune liver disease; alcoholic liver disease (ALD); non-alcoholic fatty liver diseases (NAFLD or NASH); drug-induced / toxic liver injury; cirrhosis; liver cancer; hepatolenticular degeneration; α1-antitrypsin deficiency; hemochromatosis, etc.

In addition, intrahepatic fat accumulation is an important factor in the occurrence and progression of non-alcoholic fatty liver diseases (NAFLD or NASH), and therefore a decrease in ALT and/or AST level and a reduction in liver weight after drug interventions can indicate that there are some therapeutic improvement effects with the drug.

Non-alcoholic fatty liver disease (NAFLD) is a condition in which excess fat accumulates in the liver in the form of triglycerides (TG) (steatosis). There are also patients with NAFLD who have hepatocyte injury and inflammation (steatohepatitis) in addition to excess fat, i.e. non-alcoholic steatohepatitis (NASH). NASH is widely recognized as a hepatic manifestation of metabolic syndrome, including such as type II diabetes, insulin resistance, central obesity, hyperlipemia (low HDL cholesterol, hypertriglyceridemia) and hypertension.

Hepatorenal Function Disease

Hepatorenal function disease refers to functional acute renal failure occurring in severe liver disease. In decompensated cirrhosis, hepatorenal syndrome may develop due to factors such as insufficient effective circulating blood volume and decreased prostaglandin synthesis.

Cardiovascular Disease

Triglycerides (TG) primarily participate in energy metabolism in the body, producing heat. High levels of TG in the blood can lead to increased blood viscosity, causing lipids to be deposited on the vascular wall and gradually forming small plaques, known as atherosclerosis. Increased LDL-C is a major, independent risk factor for the occurrence and progression of of atherosclerosis; increased level of LDL-C is also an indicator of coronary heart disease. As HDL-C can transport cholesterol in the blood vessel wall to the liver for catabolism (i.e. reverse cholesterol transport), it can reduce cholesterol deposition in the vascular wall and play an anti-atherosclerotic role.

Inflammations

Lipopolysaccharide (LPS), also known as endocytotoxin, is a phospholipid that forms the outer cell wall of Gram-negative bacteria. In addition to ensuring the structural integrity of the bacteria, lipopolysaccharide protects these bacteria from decomposition by bile salts secreted by the gallbladder. Typically, lipopolysaccharide is blocked from blood flow by the tight junctions of intestinal wall cells. If lipopolysaccharide enters the bloodstream, it induces a strong inflammatory response in the animal. So lipopolysaccharide levels in the blood can reflect inflammation levels.

Obesity

Obesity refers to a condition of excessive body weight and thickened fat layer, resulting from excessive accumulation of body fat, especially triglycerides, which represents an abnormal or excessive accumulation of fat that poses substantial health risks. Excessive accumulation of body fat due to excessive food intake or changes in body metabolism results in excessive weight gain and pathological or physiological changes or latent conditions in the body. A Body mass index of over 25 is considered overweight and over 30 is considered obesity. Obesity increases the risk of various physical and mental diseases. It is primarily associated with ametabolic syndrome, including a combination of type II diabetes, hypertension, hypercholesterolemia, hypertriglyceridemia and the like. In general, obesity-related health effects fall into two major categories: diseases attributable to increased body fat (e.g., osteoarthritis, obstructive sleep apnea, etc.) and diseases linked to increased number of adipocytes (e.g., diabetes, dyslipidemia, cancer, cardiovascular disease, non-alcoholic fatty liver disease or non-alcoholic steatohepatitis, etc.).

The "obesity-related disease" may be selected from the following diseases: overeating, binge eating, buimia, hypertension, diabetes, elevated plasma insulin concentrations, insulin resistance, hyperlipidemia, metabolic syndrome, insulin resistance syndrome, obesity-associated gastroesophageal reflux disease, atherosclerosis, hypercholesterolemia, hyperuricemia, low back pain, cardiac hypertrophy and left ventricular hypertrophy, lipid metabolism disorders, non-alcoholic steatohepatitis, cardiovascular disease, and polycystic ovary syndrome, as well as those subjects with these obesity-related diseases, including those who wish to lose weight.

Obesity-related diseases in the present application include at least one of the following diseases: obesity, metabolic syndrome, cardiovascular disease, hyperlipidemia, hypercholesterolemia, hypertension, insulin-resistant syndrome, obesity-associated gastroesophageal reflux disease, and steatohepatitis.

Peptide YY (PYY), also known as tyrosine-tyrosine peptide, is a gastrointestinal peptide hormone produced by L-cells located in the jejunum, ileum, and colon, and PYY secretion is stimulated by food intake (mainly fat). At least five different Y receptor subtypes of the GPCR family (Y1, Y2, Y4, Y5, and Y6) are peptide YY receptors in the digestive tract, pancreas and central nervous system. The primary function of peptide YY is to reduce food intake, inhibit gastric emptying and secretion, and inhibit intestinal motility and secretion of electrolyte and pancreatic.

Secretin, also known as pancreatotrophin, is secreted by almost all enteroendocrine cells, but mainly by S cells located in the duodenal mucosa. Acidic chyme and digestion of fats and proteins stimulate secretion of secretin. The main functions of secretin are to stimulate the secretion of alkaline substances from the pancreas and biliary system, to inhibit gastric motility and gastric acid secretion, as well as to participate in the homeostatic/osmotic regulation of body fluids. Secretin receptors (B-class GPCR) are located on the membranes of pancreatic ductal cells and centroacinar cells, on epithelial cells of large intrahepatic bile ducts, and in the kidney.

Glucagon-like peptide-1 (GLP-1) is a hormone produced primarily by intestinal L-cells and belongs to the incretin family. Glucagon-like peptide-1 receptor agonist (GLP-1RA) is a novel type of antidiabetic drugs in recent years, which enhances insulin secretion and inhibits glucagon secretion by activating the GLP-1 receptor in a glucose-dependent manner and is able to delay gastric emptying and reduce the amount of food intake through central appetite suppression, thus achieving blood glucose lowering, weight loss and other effects.

Glucose-dependent insulinotropic release polypeptide (GIP), a 42-amino acid peptide, is produced by intestinal K-cells located primarily in the duodenum and proximal jejunum. GIP, like GLP-1, has a short half-life (4-7 minutes). GIP was the first incretin to be discovered, and GIP accounts for about 2/3 of the incretin effect in humans, higher than GLP-1. Endogenous GIP stimulates glucose-dependent insulin secretion and has a stronger incretin effect than GLP-1. GIP has a dual function in response to glucagon, with the ability to stimulate glucagon secretion in the normoglycemic and hypoglycemic states, but inhibit the glucagon secretion in the hyperglycemic state.

Leptin is a hormone secreted by adipose tissue, and its content in serum is in proportion to the size of adipose tissue in the animal. Leptin acts on receptors located in the central nervous system to regulate the behavior and metabolism of organisms. When an animal body has reduced body fat or is in a low-energy state (e.g., starvation), serum level of leptin decreases significantly, which stimulates foraging behavior of animial while reducing its own energy expenditure. Conversely, when an organism's body fat increases, serum levels of leptin are elevated, which inhibits food intake and accelerates metabolism. It is through such negative feedback mechanisms that leptin regulates the energy balance of an organism as well as the body weight.

As fat mass continues to increase, leptin continues to be secreted, and the long-term stimulation of large amounts of leptin makes the brain less sensitive to leptin, which is medically known as "leptin resistance".

Resistin is a hormone or adipokine secreted by adipose tissue and is associated with obesity and insulin resistance. In humans, resistin has been characterized as a hormone expressed and secreted by immune cells, particularly macrophages, and has been associated with many inflammatory responses, including inflammation of adipose tissue caused by macrophage infiltration. Resistin can play an important role in the occurrence and development of obesity and insulin resistance through resistin-induced inflammation. Resistin has also been associated with other chronic diseases, such as cardiovascular disease and cancer, and has been proposed as an important biomarker for metabolism-related diseases in many studies.

Short-chain fatty acids are one of the important metabolites produced by intestinal microorganism and act as signaling molecules to influence a range of host activities, primarily acetate, propionate and butyrate. Short-chain fatty acids may lower intestinal pH and inhibit pathogen growth. Short-chain fatty acids can activate target pathways such as GPR41, GPR43, GPR109A, and GPCR81, improve the integrity and function of colonic epithelial cells, enhance the intestinal barrier function, promote the secretion of GLP-1, PYY, and other hormones in the enteroendocrine cells, increase the sensitivity of insulin, increase the energy expenditure, promote lipolysis, inhibit the production of pro-inflammatory cytokines, and maintain intestinal immune homeostasis. Short-chain fatty acids have beneficial effects on metabolic diseases such as obesity, diabetes, non-alcoholic fatty liver, non-alcoholic steatohepatitis, as well as ulcerative colitis, radiation proctitis and Crohn's disease.

Propionic acid and butyric acid can act as HDAC inhibitors. The concentration of butyric acid (mM) in the intestinal lumen is high and butyric acid is the main energy source for colonocytes to maintain the integrity of the intestinal barrier function. Butyric acid can partly alter the expression of multiple functional genes through the inhibition of HDAC, and modulate cell proliferation, apoptosis, and differentiation, thereby inhibiting the occurrence and development of colorectal cancer and inflammation.

Secretin is a polypeptide of 27 amino acids that is secreted by duodenal S cells and belongs to the glucagon superfamily. Secretin acts on the central amygdala through the cyclic adenosine monophosphate-protein kinase A (cAMP-PKA) pathway to reduce food intake and lower blood glucose. Recent studies have found that secretin levels increase after food intake, and its binding to the secretin receptor (SCTR) in brown adipose tissue (BAT) to induce lipolysis, which promotes thermogenesis in BAT, leading to central satiety through the gut-secretin-BAT-brain axis, which in turn reduces food intake. The study also found that levels of plasma glucose and triglyceride were lower, and glucose tolerance and insulin resistance were significantly improved in secretin receptor knockout mice (SCTR-/-) fed a high-fat diet, compared to wild-type mice. In diabetic patients, plasma secretin levels are reduced. Altered secretin levels stimulate renal secretin receptors, contributing to polydipsia and polyuria symptoms in diabetic patients.

The technical solutions of the present invention will be described in detail in combination with the following examples. It will be understood by those skilled in the art that the following examples are used only to illustrate the invention and should not be considered as limiting the scope of the invention. Techniques and conditions not specifically mentioned in the examples follow those described in the literature or manufacturer's instructions for products and instruments. All reagents or instruments may be purchased commercially if the manufacturer is indicated.

**Examples**

The liquid MM01 medium involved in the examples has the following components: 5 g/L of peptone, 5 g/L of trypticase, 10 g/L of yeast powder, 5 g/L of beef extract, 5 g/L of glucose, 2 g/L of $K_2HPO_4$, 2 g/L of sodium acetate, 1 mL/L of Tween 80, 5 mg/L of hemoglobin, 0.5 g/L of L-cysteine hydrochloride, 1 μL/L of vitamin K1, and 8 ml/L of inorganic salt solution (including per liter: 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride).

The solid MM01 medium involved in the examples has the following components: 5 g/L of peptone, 5 g/L of trypticase, 10 g/L of yeast powder, 5 g/L of beef extract, 5 g/L of glucose, 2 g/L of $K_2HPO_4$, 2 g/L of sodium acetate, 1 mL/L of Tween 80, 5 mg/L of hemoglobin, 0.5 g/L of L-cysteine hydrochloride, 1 $\mu$L/L of vitamin K1, and 8 ml/L of inorganic salt solution (including per liter: 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride), and 15g/L of agar.

The anaerobic blood agar plate (purchased from Huankai Microbial) involved in the examples is formulated (per liter) as follows: 10.0 g of casein tryptic digest, 3.0 g of cardiac tryptic digest, 1.0 g of corn starch, 5.0 g of meat pepsin digest, 5.0 g of yeast extract powder, 5.0 g of sodium chloride, 15.0 g of agar, 50-100 mL of sterile defibrinated sheep blood, and 1000 mL of distilled water; and final pH $7.3 \pm 0.2$.

The tryptic soy broth (TSB) liquid medium used in the examples contains per liter: 17.0 g of tryptone, 3.0 g of soybean papain hydrolysate, 2.5 g of dipotassium hydrogen phosphate, 5.0 g of sodium chloride, and 2.5 g of glucose; and pH $7.3 \pm 0.2$.

Conventional methods of preparation and sterilization may be used to prepare the above medium.

The solvent involved in the examples (also known as PBS-Cys) is a phosphate buffer containing 0.05% cysteine hydrochloride.

The primers used to amplify 16S rRNA in the examples are:

27F: AGAGTTTGATCMTGGCTCAG (SEQ ID NO.4); and
1492R: TACGGYTACCTTGTTACGACTT (SEQ ID NO.5).

**Example 1. Isolation and Identification of the Strains**

1.1 Isolation and sequencing of strain MNH05119

Isolation

The intestinal strain MNH05119 was isolated from a fecal sample of a healthy volunteer from Guangzhou City, Guangdong Province, China. The donor self-collected 2 to 5 grams of fresh feces into sample collection and preservation tubes. After homogenization by vortexing, the processed fecal samples was stored in an ice box and delivered to the laboratory for strain isolation within 24 hours.

Specifically, the fresh fecal sample were placed in an anaerobic workstation (Don Whitley Scientific H35), vortexed for 1 min to ensure homogeneity using a vortex shaker, and 1 mL of the sample was transferred into 9 mL of saline, mixed as a $10^{-1}$ dilution, which was subsequently diluted to $10^{-6}$ for later use. $10^{-6}$ dilution was dropped on the isolation medium anaerobic blood agar plate (Huankai Microbial Technology Co., Ltd.), the amount of drops was 100 $\mu$L/plate, coated evenly, and after the surface of the plate was dry, the plate was inverted and incubated at 37 °C for 3 to 5 days. Strain growth was observed and single colony was picked with a sterilized toothpick for strain purification. Purified strains were cultured anaerobically on anaerobic blood agar plates at 37°C. Purified culture strains were stored in 20% (W/V) glycerol at -86°C of low temperature.

Culture and Morphological Characterization

Strain MNH05119 was inoculated into medium MM01 and cultured anaerobically at 37°C for 48 to 96 h. Visible colonies formed on the medium were observed.

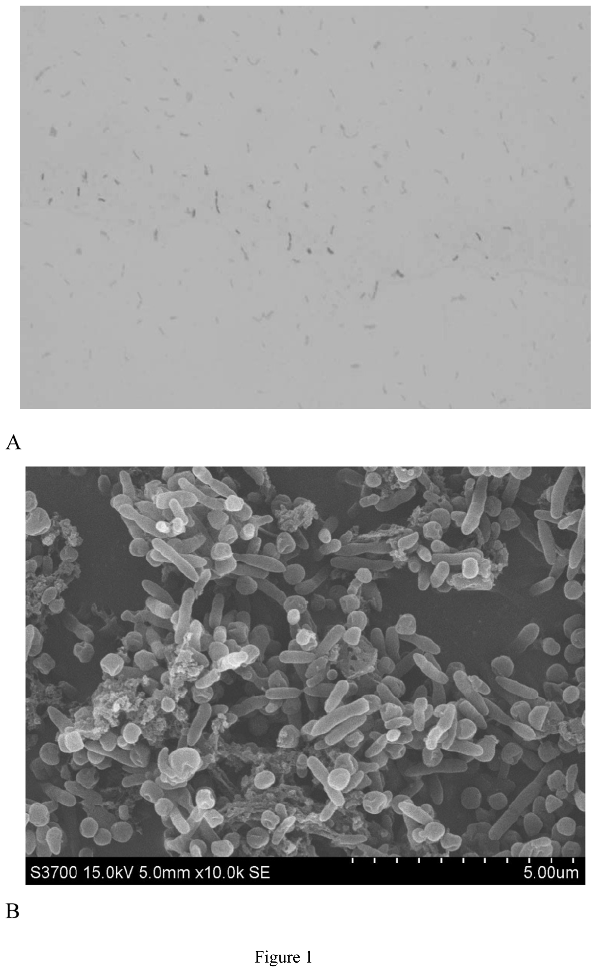

Colonies were light yellow, round, opaque, with moist surface and neat edges, about 0.5 to 5 mm in diameter. Microscopic morphologic examination revealed that the cells were negative for Gram staining, non-spore-forming, lacked flagella, non-motile, rod-shaped or short-rod-shaped. The specific morphologies are shown in Figures 1A and 1B.

16S rRNA Gene Sequence

The 16S rRNA gene sequence of strain MNH05119 was amplified and sequenced for strain identification.

16S rRNA sequence of strain MNH05119 (SEQ ID NO.1):

AGATGCGAAGCATCGAGCAACACGCGAAAAAAGAGCTAACACGAGGAAGGAAAGA
AGTGAGTATTGGGTGCAGACCGCTAGACCGAGTGGCGGACGGGTGAGTAACGCGTGA
GCAACCTGCCCTGCAACGGGGGACAACAGTTGGAAACGACTGCTAATACCGCATAAGA
CCACGGTACCGCATGGTACAGGGGTAAAAGGATTTATTCGATGCAGGATGGGCTCGCGT
CCCATTAGATAGTTGGTGAGGTAACGGCCCACCAAGTCAACGATGGGTAGCCGACCTG
AGAGGGTGATCGGCCACACTGGAACTGAGACACGGTCCAGACTCCTACGGGAGGCAG
CAGTGGGGAATATTGGGCAATGGGGGAAACCCTGACCCAGCAACGCCGCGTGAGGGA
AGAAGGTCTTCGGATTGTAAACCTTTGTCCTATGGGACGAAACAAATGACGGTACCATA
GGAGGAAGCTCCGGCTAACTACGTGCCAGCAGCCGCGGTAATACGTAGGGAGCAAGCG
TTGTCCGGAATTACTGGGCGTAAAGGGTGCGTAGGTGGTCATGTAAGTCAGATGTGAA
AGACCGGGGCTTAACCCCGGGATTGCATTTGAAACTGTGTGACTTGAGTACAGGAGAG
GGAAGTGGAATTCCTAGTGTAGCGGTGAAATGCGTAGATATTAGGAGGAACACCAGTG
GCGAAGGCGACTTTCTGGACTGTAACTGACACTGAAGCACGAAAGCGTGGGGAGCAA
ACAGGATTAGATACCCTGGTAGTCCACGCCGTAAACGATGGATACTAGGTGTGGGGCCC
GATAGGGTTCCGTGCCGAAGCTAACGCATTAAGTATCCCGCCTGGGGAGTACGATCGCA
AGGTTGAAACTCAAAGGAATTGACGGGGGCCCGCACAAGCAGCGGAGCATGTGGTTT
AATTCGAAGCAACGCGAAGAACCTTACCAAGGCTTGACATCGTCTGACGACTGTAGAG
ATACAGTTTCCCTTCGGGGCAGACAGACAGGTGGTGCATGGTTGTCGTCAGCTCGTGTC
GTGAGATGTTGGGTTAAGTCCCGCAACGAGCGCAACCCTTATTGCTAGTTGCCAGCACG
TAAAGGTGGGAACTCTAGTGAGACTGCCGGGGACAACTCGGAGGAAGGTGGGGACGA
CGTCAAATCATCATGCCCCTTATGTCTTGGGCTACACACGTGCTACAATGGCCGGTACA
AAGGGCAGCGAACCCGCAAGGGGAAGCGAATCTCAAAAAGCCGGTCCCAGTTCGGAT
TGTGGGCTGCAACCCGCCCACATGAAGTCGGAGTTGCTAGTAATCGCGAATCAGCATGT
CGCGGTGAATGCGTTCCCGGGCCTTGTACACACCGCCCGTCACACCACGGAAGTTGGG
AGCACCCGAAGCCAGTGGCTTAACCGTAAGGAGAGAGCTT.

1.2 Isolation and Sequencing of Strain MNH06163

Isolation

The intestinal strain MNH06163 was isolated from a fecal sample of a healthy volunteer from Guangzhou City, Guangdong Province, China. The donor self-collected 2 to 5 grams of fresh feces into sample collection and preservation tubes. After homogenization by vortexing, the processed fecal samples was stored in an ice box and delivered to the laboratory for strain isolation within 24 hours. The specific separation method followed the same procedure as mentioned above.

Culture and Morphological Characterization

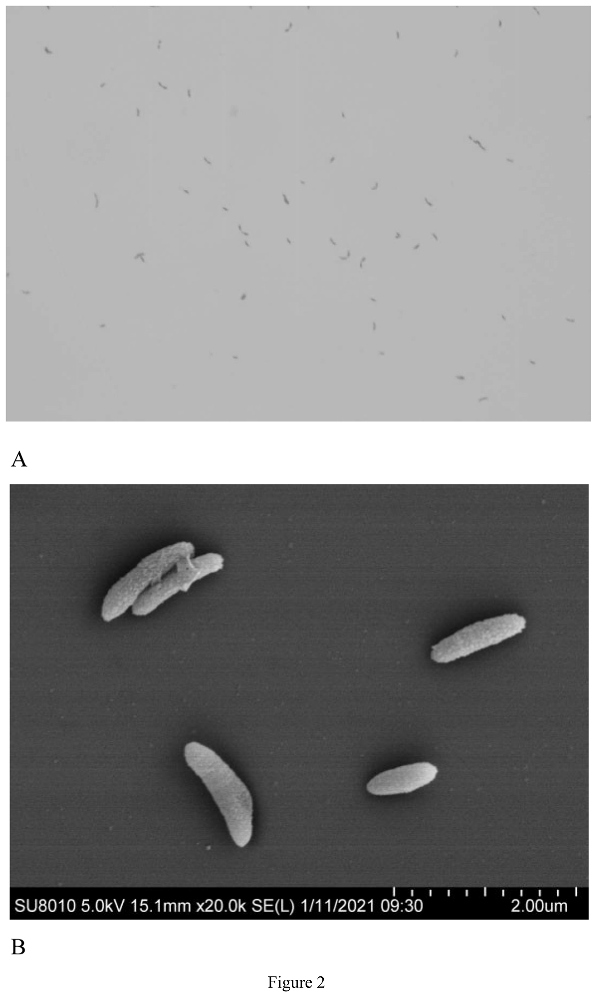

Strain MNH06163 was inoculated into medium MM01 and cultured anaerobically at 37°C for 48 to 96 h. Visible colonies formed on the medium were observed. Colonies were light yellow, round, opaque, with moist surface and neat edges, about 0.5 to 5 mm in diameter. Microscopic morphologic examination revealed that the cells were negative for Gram staining, non-spore-forming, lacked flagella, non-motile, fusiform-shaped or rod-shaped. Electron microscopic observation showed that the cells were 0.3-0.4 $\mu$m×0.7-1.4 $\mu$m, and were either single or arranged in pairs. The specific morphologies are shown in Figures 2A and 2B.

16S rRNA Gene Sequence

The 16S rRNA gene sequence of strain MNH06163 was amplified and sequenced for strain identification.

16S rRNA sequence of strain MNH06163 (SEQ ID NO.2):

TCGAACGAGGGTCATACGCGTGAACTGCACCGAGTACTTACGAAAGACCGGAGTGAA

AGCGAAGCGTTTTTGCGAGATGCGAAGCATCGAGCAACACGCGAAAAAAGAGCTAA

CACGAGGAAGGAAAGAAGTGAGTATTGGGTGCAGACCGCTAGACCGAGTGGCGGAC

GGGTGAGTAACGCGTGAGCAACCTGCCCTGCAACGGGGGACAACAGTTGGAAACGA

CTGCTAATACCGCATAAGACCACGGTACCGCATGGTACAGGGGTAAAAGGATTTATTCG

ATGCAGGATGGGCTCGCGTCCCATTAGATAGTTGGTGAGGTAACGGCCCACCAAGTCA

ACGATGGGTAGCCGACCTGAGAGGGTGATCGGCCACACTGGAACTGAGACACGGTCC

AGACTCCTACGGGAGGCAGCAGTGGGGAATATTGGGCAATGGGGGAAACCCTGACCC

AGCAACGCCGCGTGAGGGAAGAAGGTCTTCGGATTGTAAACCTTTGTCCTATGGGAC

GAAACAAATGACGGTACCATAGGAGGAAGCTCCGGCTAACTACGTGCCAGCAGCCGC

GGTAATACGTAGGGAGCAAGCGTTGTCCGGAATTACTGGGCGTAAAGGGTGCGTAGGT

GGTCATGTAAGTCAGATGTGAAAGACCGGGGCTTAACCCCGGGATTGCATTTGAAACT

GTGTGACTTGAGTACAGGAGAGGGAAGTGGAATTCCTAGTGTAGCGGTGAAATGCGT

AGATATTAGGAGGAACACCAGTGGCGAAGGCGACTTTCTGGACTGTAACTGACACTG

AAGCACGAAAGCGTGGGGAGCAAACAGGATTAGATACCCTGGTAGTCCACGCCGTAA

ACGATGGATACTAGGTGTGGGGCCCGATAGGGTTCCGTGCCGAAGCTAACGCATTAAG

TATCCCGCCTGGGGAGTACGATCGCAAGGTTGAAACTCAAAGGAATTGACGGGGGCC
CGCACAAGCAGCGGAGCATGTGGTTTAATTCGAAGCAACGCGAAGAACCTTACCAAG
GCTTGACATCGTCTGACGACTGTAGAGATACAGTTTCCCTTCGGGGCAGACAGACAGG
TGGTGCATGGTTGTCGTCAGCTCGTGTCGTGAGATGTTGGGTTAAGTCCCGCAACGAG
CGCAACCCTTATTGCTAGTTGCCAGCACGTAAAGGTGGGAACTCTAGTGAGACTGCCG
GGGACAACTCGGAGGAAGGTGGGGACGACGTCAAATCATCATGCCCCTTATGTCTTGG
GCTACACACGTGCTACAATGGCCGGTACAAAGGGCAGCGAACCCGCAAGGGGAAGCG
AATCTCAAAAAGCCGGTCCCAGTTCGGATTGTGGGCTGCAACCCGCCCACATGAAGTC
GGAGTTGCTAGTAATCGCGAATCAGCATGTCGCGGTGAATGCGTTCCCGGGCCTTGTA
CACACCGCCCGTCACACCACGGAAGTTGGGAGCACCCGAAGC.

### 1.3 Isolation and Sequencing of Strain MNH04863

The isolation and identification of strain MNH04863 and the sequences obtained can be found in the Chinese invention patent application publication CN113069475A. Specifically, after anaerobic cultivation at 37°C for 72h, the colonies of this strain were light yellow, round, with moist surface, translucent, and neat edges. The cells were short rod-shaped, non-spore-forming, lacked flagella, non-motile, 0.3-0.4 $\mu$m$\times$0.6-1.1 $\mu$m, singly or in pairs, and negative for Gram staining.

16S rRNA sequence of strain MNH04863MNO-863 (SEQ ID NO.3):

TCGAACGAAGTTGCTCTTTGTGAAGCCCTCGGGTGGAACTGCGAGTATACTTAGTG
GCGGACGGGTGAGTAACGCGTGAGCAATCTGCCCTGCAATGGGGGACAACAGTTGGA
AACGACTGCTAATACCGCATGAGACCACGAAACCGCATGGTTTTGAGGTAAAAGGATTT
ATTCGATGCAGGATGAGCTCGCGTCCCATTAGATAGTTGGTGAGGTAACGGCCCACCAA
GTCAACGATGGGTAGCCGACCTGAGAGGGTGATCGGCCACACTGGAACTGAGACACG
GTCCAGACTCCTACGGGAGGCAGCAGTGGGGAATATTGGGCAATGGGGGAAACCCTGA
CCCAGCAACGCCGCGTGAGGGAAGAAGGTCTTCGGATTGTAAACCTTTGTCCTATGGG
ACGAAACAAATGACGGTACCATAGGAGGAAGCTCCGGCTAACTACGTGCCAGCAGCCG
CGGTAATACGTAGGGAGCAAGCGTTGTCCGGAATTACTGGGCGTAAAGGGTGCGTAGG
TGGCTATGTAAGTCAGATGTGAAAGACCGGGGCTTAACCCCGGGGTTGCATTTGAAACT
GTGTGGCTTGAGTACAGGAGAGGGAAGTGGAATTCCTAGTGTAGCGGTGAAATGCGTA
GATATTAGGAGGAACACCAGTGGCGAAGGCGACTTTCTGGACTGTAACTGACACTGAA
GCACGAAAGCGTGGGGAGCAAACAGGATTAGATACCCTGGTAGTCCACGCCGTAAACG
ATGGATACTAGGTGTGGGGCCCGATAGGGTTCCGTGCCGAAGCTAACGCATTAAGTATC
CCGCCTGGGGAGTACGATCGCAAGGTTGAAACTCAAAGGAATTGACGGGGGCCCGCA

CAAGCAGCGGAGCATGTGGTTTAATTCGAAGCAACGCGAAGAACCTTACCAAGGCTTG

ACATCCTCTGACGACTGTAGAGATACAGTTTCCCTTCGGGGCAGAGAGACAGGTGGTG

CATGGTTGTCGTCAGCTCGTGTCGTGAGATGTTGGGTTAAGTCCCGCAACGAGCGCAA

CCCTTATTGCTAGTTGCCAGCGCGTAAAGGCGGGAACTCTAGTGAGACTGCCGGGGAC

AACTCGGAGGAAGGTGGGGACGACGTCAAATCATCATGCCCCTTATGTCTTGGGCTAC

ACACGTGCTACAATGGCCGGTACAAAGGGCAGCGAACCCGTAAGGGGAAGCGAATCT

CAAAAAGCCGGTCCCAGTTCGGATTGTGGGCTGCAACCCGCCCACATGAAGTCGGAGT

TGCTAGTAATCGCGAATCAGCATGTCGCGGTGAATGCGTTCCCGGGCCTTGTACACACC

GCCCGTCACACCACGGAAGTTGGGAGCACCCGAAGCCAGTAGG.

1.4 Identification of Strains MNH05119, MNH06163, and MNH04863

Strains MNH05119 and MNH06163 were analyzed for 16S rRNA to preliminarily determine their taxonomic status.

Specifically, the 16S rRNA sequences of strains MNH05119 and MNH06163 were aligned with the NCBI 16S rRNA sequence database. The closest species for both strains was *Christensenella minuta,* with a similarity of 98.56%. the 16S rRNA sequences of MNH04863 were aligned with the NCBI 16S rRNA sequence database and the closest species was *Christensenella intestinihominis,* with a similarity of 100%. Based on these findings, it was preliminarily determined that strains MNH05119, MNH06163, and MNH04863 belong to the *Christensenella.*

When the identity between the 16S rRNA gene sequences of two strains is less than 98.65%, they can be judged to belong to different species (see, Kim, M., Oh, H.-S., Park, S.-C., & Chun, J. (2014). Towards a taxonomic coherence between average nucleotide identity and 16S rRNA gene sequence similarity for species demarcation of prokaryotes. International Journal of Systematic and Evolutionary Microbiology, 64(Pt 2), 346-351, and Liu, C., Du, M.-X., Abuduaini, R., Yu, H.-Y., Li, D.-H., Wang, Y.-J., Liu, S.-J. (2021). Enlightening the taxonomy darkness of human gut microbiomes with a cultured biobank. Microbiome, 9(1), p.23). Therefore, it can be determined that strains MNH05119 and MNH06163, although belonging to the *Christensenella,* are not *Christensenella minuta.*

Further, for accurate taxonomic identification of the strains, whole genome sequencing was performed for strains MNH05119, MNH06163, and MNH04863. The DNA samples of the species were randomly fragmented by ultrasonic cell disruptor; and then the library preparation was completed by the steps of end-repair, poly-A tailing, addition of sequencing adapters, purification, PCR amplification, and other steps, and then PE150 paired-end sequencing was carried out using Illunima Novaseq 6000 platform.

Genome-wide information GCA_900155415.1 for *Christensenella massiliensis* Marseille-p2438, GCF_003628755.1 for *Christensenella minuta* DSM 22607, and GCF_900087015.1 for *Christensenella timonensis* Marseille-P2437 and GCF_001678845.1 for *Christensenella intestinihominis* AF73-05CM02 were obtained from NCBI. Using fast ANI software, the whole genome sequencing results of strain MNH04863 were aligned and analyzed with the genomic information of the above collected *Christensenella massiliensis, Christensenella minuta,* and *Christensenella timonensis.* Similarly, the whole genome sequencing results of strains MNH05119 and MNH06163 were aligned and analyzed with the genomic information of the above collected *Christensenella massiliensis, Christensenella minuta, Christensenella timonensis,* and *Christensenella intestinihominis.* The results are shown in Table 1 below.

Table 1: Whole Genome alignment Results

|  |  | Name of the reference strain | Sequence of the reference strain | ANI | AF |
|---|---|---|---|---|---|
|  | MNH04863 | Christensenella minuta DSM 22607 | GCF_003628755.1_ASM362875v1_ genomic.fna | 83.18% | 65.80% |
|  | MNH04863 | Christensenella timonensis Marseille-P2437 | GCF_900087015.1_PRJEB13910_g enomic.fna | 76.80% | 38.37% |
|  | MNH04863 | Christensenella massiliensis Marseille-p2438 | GCA_900155415.1 | 79.81% | 35.10% |

(continued)

|  | Name of the reference strain | Sequence of the reference strain | ANI | AF |
|---|---|---|---|---|
| MNH05119 | Christensenella intestinihominis AF73-05CM02 | GCF_001678845.1_ASM167884v1_ genomic.fna | 79.20% | 48.82% |
| MNH05119 | Christensenella minuta DSM 22607 | GCF_003628755.1_ASM362875v1_ genomic.fna | 77.60% | 45.95% |
| MNH05119 | Christensenella timonensis Marseille-P2437 | GCF_900087015.1_PRJEB13910_g enomic.fna | 76.94% | 40.06% |
| MNH05119 | Christensenella massiliensis Marseille-p2438 | GCA_900155415.1 | 78.38% | 25.3% |
| MNH06163 | Christensenella massiliensis Marseille-p2438 | GCA_900155415.1 | 78.36% | 25.6% |
| MNH06163 | Christensenella intestinihominis AF73-05CM02 | GCF_001678845.1_ASM167884v1_ genomic.fna | 79.08% | 48.62% |
| MNH06163 | Christensenella minuta DSM 22607 | GCF_003628755.1_ASM362875v1_ genomic.fna | 77.61% | 44.73% |
| MNH06163 | Christensenella timonensis Marseille-P2437 | GCF_900087015.1_PRJEB13910_g enomic.fna | 76.94% | 39.78% |

Genomic correlation analysis based on average nucleotide identity (ANI) showed that strains MNH04863, MNH05119, and MNH06163 were significantly distinct from other species of the *Christensenella.* For example, the ANI value of MNH04863 to the closest species of *Christensenella minutaas* determined by the above 16S rRNA sequence alignment was less than 83.5%, the ANI value of MNH05119 to the closest species of *Christensenella intestinihominis* determined by the above 16S rRNA sequence alignment was less than 79.2%, and the ANI value of MNH06163 to the closest species of *Christensenella intestinihominis* determined by the above 16S rRNA sequence alignment was less than 79.08%.

In the field of bacterial taxonomy, an ANI greater than 95% is usually recognized as belonging to the same species. The ANI values of the three strains MNH04863, MNH05119, and MNH06163 in the present application are significantly lower than 95% compared to existing species of *Christensenella* and it can therefore be determined that they do not belong to any existing species within the *Christensenella* but instead represented novel species of *Christensenella.*

1.5 Position of Strains MNH05119, MNH06163, and MNH04863 in the Phylogenetic Tree

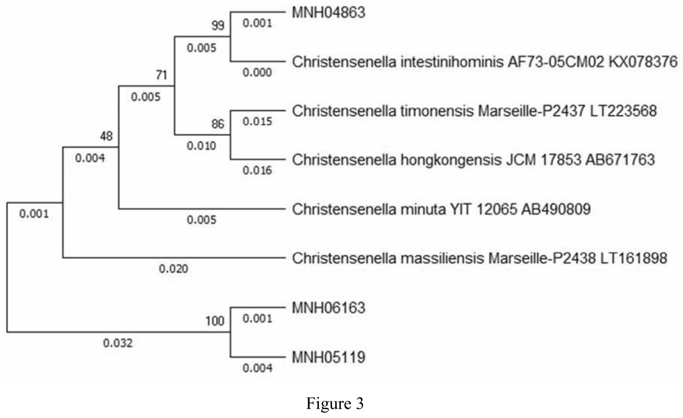

The 16S rRNA gene sequences of MNH05119, MNH06163, and MNH04863 were aligned with sequences from related strains of the genus *Christensenella* and its closely related species retrieved from databases such as GenBank. The 16S rRNA gene sequences of MNH05119, MNH06163, and MNH04863 were subsequently aligned with the 16S rRNA gene sequences of type strains that showed high sequence similarity, which were obtained from the NCBI database. Multiple sequence alignment was conducted, after which a phylogenetic tree was constructed using the maximum likelihood method through MEGA 5 software (Figure 3). In Figure 3, only Bootstrap values greater than 50% are displayed at the nodes of the phylogenetic tree. The positions of MNH05119, MNH06163, and MNH04863 in the phylogenetic tree were clearly indicated.

Therefore, it can be concluded that strains MNH05119 and MNH06163 belong to the same species within the *Christensenella.* Strain MNH04863 belongs to a different species within the *Christensenella* compared to MNH05119 and MNH06163.

**Example 2. Physiological and Biochemical Characteristics of Strains MNH05119, MNH06163, and MNH04863**

2.1 pH tolerance, NaCl tolerance, and bile salt tolerance of strains MNH05119, MNH06163, and MNH04863

Strains MNH05119 and MNH06163 had a growth temperature range of 30°C to 42°C, with an optimal growth temperature of 37°C.

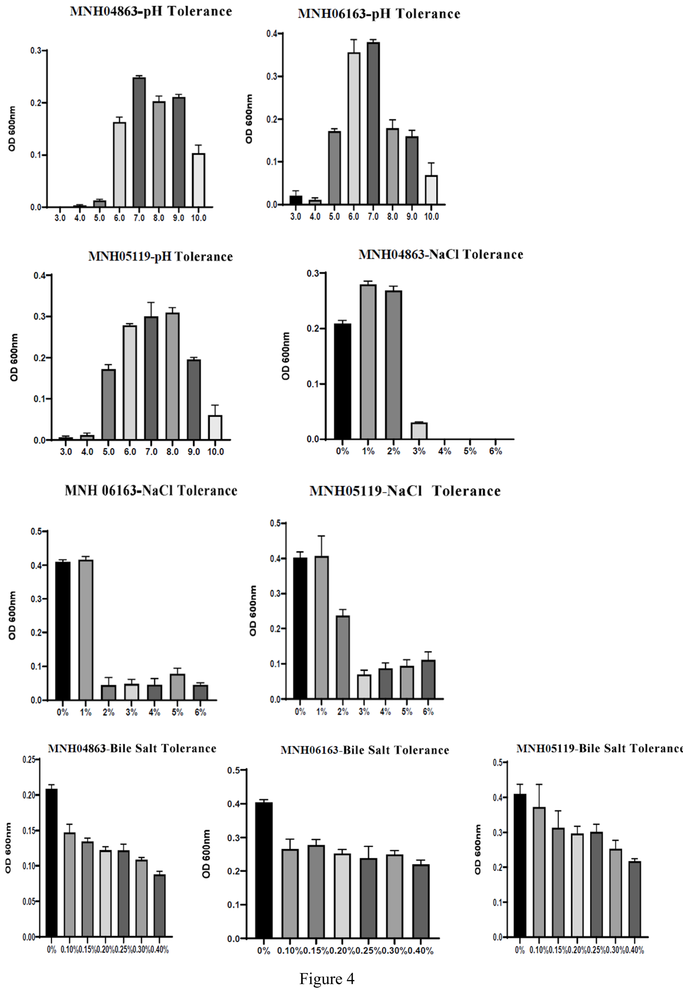

Strain MNH05119 was found to grow in the pH range of 5.0 to 9.0, with an optimal growth pH of 6.0 to 8.0. It could tolerate up to 2% NaCl; and grow at bile salt concentrations of 0% to 0.40% (Figure 4).

Strain MNH06163 was found to grow in the pH range of 6.0 to 8.0, with an optimal growth pH of 5.0 to 9.0. It could tolerate up to 1% NaCl; and grow at bile salt concentrations of 0% to 0.40% (Figure 4).

Both strains MNH05119 and MNH06163 were able to survive for 2 h under aerobic conditions with a survival rate of about 87.88%, and grew well under anaerobic conditions.

Strain MNH04863 had a growth temperature range of 30 to 42°C, with an optimal growth temperature of 37°C. It was found to grow in the pH range of 6.0 to 10.0, with an optimal growth pH of 7.0 to 9.0. It could tolerate up to 2% NaCl; and grow at bile salt concentrations of 0% to 0.15%, but it could not grow when the bile salt concentrations were greater than or equal to 0.4% (using an OD value of 0.10 as the threshold, see Figure 4). Strain MNH04863 fails to grow under aerobic conditions and grows well under anaerobic conditions, belonging to an obligate anaerobe.

2.2 API 20A Test for Strains MNH05119 and MNH06163

Tests were performed using API 20A test strips (BioMérieux) according to the instructions. The strains were cultured under anaerobic conditions at 37°C.

Table 2: API 20A Test Results for Strain MNH05119

| Test Item | Test results | Test Item | Test results |
|---|---|---|---|
| IND | Negative | ESC | Negative |
| URE | Negative | GLY | Acid Production |
| GLU | Acid Production | CEL | No acid production |
| MAN | No acid production | MNE | Acid Production |
| LAC | No acid production | MLZ | No acid production |
| SAC | No acid production | RAF | No acid production |
| MAL | Acid Production | SOR | Acid Production |
| SAL | No acid production | RHA | Acid Production |
| XYL | Acid Production | TRE | No acid production |
| ARA | Acid Production | GEL | Negative |

Table 3: API 20A Test Results for Strain MNH06163

| Test Item | Test results | Test Item | Test results |
|---|---|---|---|
| IND | Negative | ESC | Negative |
| URE | Negative | GLY | No acid production |
| GLU | Acid Production | CEL | No acid production |
| MAN | No acid production | MNE | No acid production |
| LAC | No acid production | MLZ | No acid production |
| SAC | No acid production | RAF | No acid production |
| MAL | No acid production | SOR | No acid production |
| SAL | No acid production | RHA | Acid Production |
| XYL | Acid Production | TRE | No acid production |
| ARA | Acid Production | GEL | Negative |

Meaning of abbreviations in Tables 2 and 3

| Abbreviation | Meaning/full name | Abbreviation | Meaning/full name |
|---|---|---|---|
| IND | Indole test | ESC | Esculin |

(continued)

| Abbreviation | Meaning/full name | Abbreviation | Meaning/full name |
|---|---|---|---|
| URE | Urease | GLY | Glycerol |
| GLU | Glucose | CEL | Cellose |
| MAN | Mannitol | MNE | Mannose |
| LAC | Lactose | MLZ | Melezitose |
| SAC | Sucrose | RAF | Raffinose |
| MAL | Maltose | SOR | Sorbitol |
| SAL | Salicin | RHA | Rhamnose |
| XYL | Xylose | TRE | Trehalose |
| ARA | Arabinose | GEL | Gelatine |

2.3 Antibiotic Susceptibility Testing of Strains MNH05119, MNH06163, and MNH04863

Antibiotic susceptibility testing was performed separately for each strain using the disk diffusion test. The test results are shown in Table 4. Strain MNH05119 was sensitive to antibiotics such as erythromycin, chloramphenicol, and tetracycline, and showed resistance to antibiotics such as gentamicin, penicillin, ciprofloxacin, compound sulfamethoxazole, ampicillin, lincomycin, and ceftriaxone. Strain MNH06163 was sensitive to antibiotics such as erythromycin, chloramphenicol, and tetracycline, and showed resistance to antibiotics such as gentamicin, penicillin, ciprofloxacin, compound sulfamethoxazole, ampicillin, lincomycin, and ceftriaxone. Strain MNH04863 was sensitive to antibiotics such as erythromycin, chloramphenicol, tetracycline, penicillin, lincomycin, and ceftriaxone, and showed resistance to antibiotics such as ampicillin, compound sulfamethoxazole, ciprofloxacin, and gentamicin.

Table 4: Antibiotic Susceptibility Test Results for the Strains

| Antibiotics | MNH05119 Inhibition Zone Diameter (mm) | MNH06163 Inhibition Zone Diameter (mm) | MNH04863 Inhibition Zone Diameter (mm) |
|---|---|---|---|
| Gentamycin (GEN) | 0 | 0 | 0 |
| Erythromycin (ERM) | 31.65 | 8.72 | 16.82 |
| Chloramphenicol (CLM) | 32.49 | 21.59 | 38.88 |
| Tetracycline (TET) | 24.12 | 18.14 | 46.11 |
| Penicillin (PEN) | 0 | 0 | 43.06 |
| Ciprofloxacin (CFX) | 0 | 0 | 0 |
| Compound sulfamethoxazole (T/S) | 0 | 0 | 0 |
| Ampicillin (AMP) | 0 | 0 | 0 |
| Lincomycin (LIN) | 0 | 0 | 29.86 |
| Ceftriaxone (CTR) | 0 | 0 | 37.32 |

**Example 3. Genomic analysis of strains MNH05119, MNH06163, and MNH04863**

3.1 Analysis of potential virulence genes

Potential virulence factors and related genes within the genomes were analyzed by aligning with the Virulence Factor Database (VFDB, http://www.mge.ac.cn/cgi-bin/VFs/v5/main.cgi, updated on September 19, 2019) using NCBI blastp (version 2.7.1+). The detailed alignment results were shown in Table 5.

Table 5: Potential virulence genes of the strains

| Strain gene | VFDB gene | Gene Name | Alignment consistency (%) |
|---|---|---|---|
| MNH06163_00463 | VFG000077 | clpP | 65.285 |
| MNH06163_00485 | VFG001855 | htpB | 60.227 |
| MNH06163_02205 | VFG037028 | katA | 61.826 |
| MNH05119_00391 | VFG000077 | clpP | 65.285 |
| MNH05119_00413 | VFG001855 | htpB | 60.227 |
| MNH05119_02562 | VFG037028 | katA | 61.826 |
| MNH04863_00269 | VFG037028 | katA | 63.655 |
| MNH04863_01151 | VFG000077 | clpP | 64.767 |
| MNH04863_01175 | VFG001855 | htpB | 60.985 |
| MNH04863_02839 | VFG002377 | ddhA | 60.618 |

3.2 Analysis of potential primary metabolism gene clusters

Potential primary metabolism gene clusters within the genome were analyzed using gutSMASH5 (version 1.0.0). The detailed alignment results were shown in Tables 6 to 8.

Table 6: Potential primary metabolism gene clusters in strain MNH05119

| Gene Cluster Range | Type | From | To | Most similar known gene clusters | Abbreviation | Similarity |
|---|---|---|---|---|---|---|
| Region 2.1 | Others_HGD_unassigned | 211171 | 235415 | | | |
| Region 3.1 | Rnf_complexPutrescine2 spermidine | 127869 | 158167 | Rnf complex C. sporogenes | RNF | 100% |
| Region 4.1 | OD_fatty_acids | 90035 | 122286 | | | |
| Region 4.2 | succinate2propionate | 134678 | 159617 | Glutamate to butyrate C. symbiosum | BUT | 20% |
| Region 7.1 | aminobutyrate2Butyrate | 151256 | 181072 | Aminobutyrate to butyrate C. pasteurianum | AMINOBUT | 40% |
| Region10.1 | Ech_complex | 7421 | 32156 | Ech complex T. phaeum | ECH | 85% |
| Region 13.1 | TPP_AA_metabolism | 18116 | 43496 | | | |
| Region 22.1 | porA | 1 | 20431 | porA C. sporogenes | POR | 60% |

Table 7: Potential primary metabolism gene clusters in strain MNH06163

| Gene cluster range | Type | From | To | Most similar known gene clusters | Abbreviation | Similarity |
|---|---|---|---|---|---|---|
| Region 1.1 | Ech_complex | 102567 | 127302 | Ech complex T. phaeum | ECH | 85% |
| Region 1.2 | OD_fatty_acids | 224593 | 256844 | | | |
| Region 1.3 | succinate2propionate | 269236 | 294175 | Glutamate to butyrate C. symbiosum | BUT | 20% |
| Region 1.4 | Others_HGD_unassigned | 591279 | 615523 | | | |

(continued)

| Gene cluster range | Type | From | To | Most similar known gene clusters | Abbreviation | Similarity |
|---|---|---|---|---|---|---|
| Region 1.5 | aminobutyrate2Butyrate | 801933 | 831749 | Aminobutyrate to butyrate C. pasteurianum | AMINOBUT | 40% |
| Region 1.6 | porA | 831899 | 854581 | porA C. sporogenes | POR | 60% |
| Region 2.1 | TPP_AA_metabolism | 274493 | 299819 | | | |
| Region 2.2 | Putrescine2spermidineRnf_complex | 406182 | 436480 | Rnf complex C. sporogenes | RNF | 100% |

Table 8: Potential primary metabolism gene clusters in strain MNH04863

| Gene cluster range | Type | From | To | Most similar known gene clusters | Abbreviation | Similarity |
|---|---|---|---|---|---|---|
| Region 1.1 | Others_HGD_unassigned | 95930 | 120183 | | | |
| Region 1.2 | Flavoenzyme_AA_peptides_catabolismsuccinate 2propionate | 421067 | 465963 | Glutamate to butyrate C. symbiosum | BUT | 30% |
| Region 1.3 | OD_fatty_acids | 478330 | 510607 | | | |
| Region 2.1 | TPP_AA_metabolismRn f_complex | 15267 | 65752 | Rnf complex C. sporogenes | RNF | 100% |
| Region 5.1 | Indoleacetate2scatole | 55791 | 78550 | | | |
| Region 5.2 | Putrescine2spermidinePF OR_II_pathway | 166132 | 192521 | PFOR II pathway B. thetaiotaomicron | PFORII | 100% |
| Region 6.1 | Flavoenzyme_AA_pepti des_catabolismFlavoenz yme_lipids_catabolism | 107989 | 141519 | | | |
| Region 7.1 | porAOD_fatty_acidsami nobutyrate2Butyrate | 21516 | 75090 | Aminobutyrate to butyrate C. pasteurianum | AMINOBUT | 40% |

3.3 Potential secondary metabolism gene clusters

Potential secondary metabolism gene clusters within the genome were analyzed using antiSMASH6 (version 6.0.1). The detailed alignment results were shown in Tables 9 to 11.

Table 9: Potential secondary metabolism gene clusters in strain MNH05119

| Gene cluster range | Type | From | To | Most similar known gene clusters | Similarity |
|---|---|---|---|---|---|
| Region 3.1 | RiPP-like | 164559 | 175374 | - | - |
| Region 11.1 | NRPS | 1 | 46477 | - | - |
| Region 23.1 | ranthipeptide | 1 | 14282 | - | - |

Table 10: Potential secondary metabolism gene clusters in strain MNH06163

| Gene cluster range | Type | From | To | Most similar known gene clusters | Similarity |
|---|---|---|---|---|---|
| Region 2.1 | RiPP-like | 388975 | 399790 | - | - |
| Region 3.1 | NRPS | 292998 | 341919 | - | - |
| Region 6.1 | ranthipeptide | 14451 | 36300 | - | - |

Table 11: Potential secondary metabolism gene clusters in strain MNH04863

| Gene cluster range | Type | From | To | Most similar known gene clusters | Similarity |
|---|---|---|---|---|---|
| Region 4.1 | RiPP-like | 125988 | 136800 | - | - |
| Region 6.1 | ranthipeptide | 119183 | 140956 | - | - |

3.4 Analysis of potential butyrate-producing genes

The butyrate-producing capability of the strains was evaluted by aligning their genomic sequences with a reference database containing genes involved in butyrate-producing pathways, which is described in "Vital M, Howe A C, Tiedje J M., Revealing the Bacterial Butyrate Synthesis Pathways by Analyzing (Meta) genomic Data[J]. Mbio, 2014, 5(2):1-11". NCBI blastp (version 2.7.1+) was utilized for the alignment and then the completeness of the butyrate-producing pathway was calculated, and the results are as follows:

Table 12: Potential butyrate-producing genes

| Strain gene | Butyrate-producing Pathway Name | Gene Name | Alignment consistency (%) |
|---|---|---|---|
| MNH05119_01380 | 4aminobutyrate | AbfD-Isom | 83.402 |
| MNH05119_00502 | Glutarate | HgdB | 71.582 |
| MNH05119_01368 | Pyruvate | Bcd | 81.794 |
| MNH05119_01366 | Pyruvate | EtfA | 74.631 |
| MNH05119_01367 | Pyruvate | EtfB | 79.615 |
| MNH06163_00574 | Glutarate | HgdB | 71.582 |
| MNH06163_00741 | Pyruvate | EtfA | 74.631 |
| MNH06163_00742 | Pyruvate | EtfB | 79.615 |
| MNH06163_00743 | Pyruvate | Bcd | 81.794 |
| MNH06163_00755 | 4aminobutyrate | AbfD-Isom | 83.402 |
| MNO-863_02414 | 4aminobutyrate | AbfD-Isom | 82.365 |
| MNO-863_01249 | Glutarate | HgdB | 70.241 |
| MNO-863_02400 | Pyruvate | Bcd | 81.794 |
| MNO-863_02398 | Pyruvate | EtfA | 74.926 |
| MNO-863_02399 | Pyruvate | EtfB | 79.231 |

The completeness of the butyrate-producing pathway was calculated to be 42.86% for strain MNH05119 and 42.86% for strain MNH06163.

It can exert an effect in treatment of metabolic diseases such as diabetes and obesity through the butyrate pathway, and it can also play a role in inhibiting histone deacetylase (HDAC) activity for the prevention or treatment of diseases mediated by HDAC activity. Inhibition of HDAC has been proposed to treat diabetes through a variety of mechanisms, including inhibition of Pdxl (Park et al., 2008, J Clin Invest, 118, 2316-24), and enhancement of the expression of the transcription factor Ngn3 to increase endocrine cell mass. HDAC inhibition is also used as a promising treatment for advanced diabetic complications such as diabetic nephropathy and retinal ischemia (Christensen et al., 2011, Mol Med, 17(5-6), 370-390).

**Example 4. Determination of Short-Chain Fatty Acids (SCFAs) of Strains**

4.1 Preparation of Bacterial Cultures

The strains MNO04863, MNH05119, and MNH06163 were inoculated in TSB liquid medium and anaerobically cultured at 37°C for 48 h. The bacterial cultures were collected by centrifugation and stored at -86°C of low temperature for use.

4.2 Preparation of Standard Solutions

Standards of acetic acid, propionic acid, butyric acid, isobutyric acid, valeric acid, isovaleric acid, and hexanoic acid were weighed, and dissolved in ethyl acetate to prepare eight standard concentration gradients: 0.1 $\mu$g/mL, 0.5 $\mu$g/mL, 1 $\mu$g/mL, 5 $\mu$g/mL, 10 $\mu$g/mL, 20 $\mu$g/mL, 50 $\mu$g/mL, and 100 $\mu$g/mL.

For each standard, 600 $\mu$L of the standard solution was taken, and added with 25 $\mu$L of 4-methylvaleric acid at a final concentration of 500 $\mu$M as an internal standard. After mixing well, the mixture was then transferred into a sample vial for GC-MS detection with an injection volume of 1 $\mu$L and a split ratio of 10:1 under split injection mode.

4.3 Metabolite Extraction

The samples stored at -86°C in section 4.1 were thawed on ice. 80 mg of sample was transferred to 2 mL glass centrifuge tube, and 900 $\mu$L of 0.5% phosphoric acid solution was added to resuspend the sample, vortexed mixing for 2 min, and centrifuged at 14,000 g for 10 min. 800 $\mu$L of the supernatant was collected and extracted with an equal volume of ethyl, vortexed mixing for 2 min, and centrifuged at 14000 g for 10 min. 600 $\mu$L of the upper organic phase was collected, and added with 4-methylvaleric acid at a final concentration of 500 $\mu$M as an internal standard. After mixing well, the mixture was then transferred into a sample vial for GC-MS detection with an injection volume of 1 $\mu$L and a split ratio of 10:1 under split injection mode.

4.4 Sample Detection and Analysis

The extracted samples in section 4.3 were separated using a gas chromatography system equipped with an Agilent DB-WAX capillary column (30 m $\times$ 0.25 mm ID $\times$ 0.25 $\mu$m). Specific procedures: initial temperature of 90°C, increasing to 120°C at 10°C/min, then to 150°C at 5°C/min, and finally to 250°C at 25°C/min, maintained for 2 min. Helium was used as the carrier gas at a flow rate of 1.0 mL/min.

An Agilent 7890A/5975C GC-MS system was used for mass spectrometry analysis. The temperature of the inlet port was 250°C, the temperature of the ion source was 230°C, the temperature of the transfer line was 250°C, and the temperature of the quadrupole was 150°C. Electron ionization (EI) source, full-scan and selected ion monitoring (SIM) modes were employed with electron energy 70 eV.

MSD ChemStation software was used to extract the chromatographic peak areas and retention times. The standard curve was plotted and the content of short-chain fatty acids in the sample extracted in 4.3 was calculated and the results are shown in Table 13.

Table 13: Short Chain Fatty Acid (SCFA) yield results of the strains

| SCFA yield ($\mu$g/g) | Acetic acid | Butyric acid | Isovaleric acid | Valeric acid | Hexanoic acid | Propanoic acid | Isobutyric acid |
|---|---|---|---|---|---|---|---|
| MNH05119 | 311.65 | 53.93 | 18.86 | 0.42 | 0.508 | 0.795 | 12.33 |
| MNH06163 | 420.35 | 71.41 | 14.62 | 0.08 | 0.259 | 0.597 | 9.66 |
| MNH04863 | 2694.8 | 360.3 | 0.845 | 0.19 | 4.18 | 5.60 | 1.14 |

As shown in the detection results, strains MNH04863, MNH05119, and MNH06163 can synthesize short-chain fatty acids such as acetic acid and butyric acid in large quantities during their growth. Short-chain fatty acids are one of the important metabolites produced by intestinal microbiota and act as signaling molecules to influence a range of activities in the host. The short-chain fatty acids can lower intestinal pH and inhibit the growth of pathogens. Short-chain fatty acids can activate target pathways such as GPR41, GPR43, GPR109A, and GPCR81, improve the integrity and function of colonic epithelial cells, enhance the intestinal barrier function, promote the secretion of GLP-1, PYY, and other hormones in the enteroendocrine cells, increase the sensitivity of insulin, increase the energy expenditure, promote lipolysis, inhibit the production of pro-inflammatory cytokines, and maintain intestinal immune homeostasis. Short-chain fatty acids have beneficial effects on obesity, diabetes, non-alcoholic fatty liver disease, non-alcoholic steatohepatitis and other metabolic diseases, as well as ulcerative colitis, radiation proctitis, Crohn's disease, colorectal cancer, asthma and the like.

Propionic acid and butyric acid are also used as HDAC inhibitors. The concentration of butyric acid (mM) in the intestinal lumen is high and butyric acid is the main energy source for colonocytes to maintain the integrity of the intestinal barrier function. Butyric acid can partly alter the expression of multiple functional genes through the inhibition of HDAC, and modulate cell proliferation, apoptosis, and differentiation, thereby inhibiting the occurrence and development of colorectal cancer and inflammation.

### Example 5. Effects of MNH05119 and MNH06163 on Liver and Kidney Functions and Related Diseases in a High-Fat Diet-induced Obesity Mouse Model

A high-fat diet-induced obesity mouse model was utilized to test the effects of strains MNH05119 and MNH06163 in improving liver and kidney function and related diseases. This experiment had been reviewed and approved by the Animal Management and Use Committee of Moon Biotech.

5.1 Experiment Methods:

1) Experimental animals: The experimental mice were C57BL/6J mice, aged 5-6 weeks, 18 mice in total, purchased from GuangDong GemPharmatech Co., Ltd.

2) Test strain: The glycerol stock of the strain MNH05119 was thawed at 37°C and inoculated on MM01 plates for activation in an anaerobic workstation. The activated strain was inoculated in MM01 liquid medium and cultured anaerobically to obtain sufficient amount of culture. This culture was centrifuged, concentrated and resuspended in a solvent (PBS-Cys: 8.0 g/L of NaCl, 0.2 g/L of KCl, 1.44 g/L of $Na_2HPO_4$, 0.24 g/L of $KH_2PO_4$, and 0.5 g/L of L-Cys-HCl) to obtain the subjects with purity and viable bacterial counts ($1 \times 10^{11}$ CFU/mL) that satisfy the requirements of the animal experiments.

3) Negative control (HFD-control group): PBS-Cys was used as a negative control.

4) Experimental procedure: 5 to 6-week-old C57BL/6J male mice were fed with high-fat diet for 10 weeks after quarantine. 18 mice with body weight ranging from 35.50g-44.49g were selected and randomly grouped according to their body weights, with 9 mice/group divided into 2 groups (experimental group and control group). Intragastric administration was started after grouping (Day1), and MNH05119 was administered in the experimental group and the negative control substance was administered in the control group. Each group was administered twice daily at a dose of 0.2 mL per administration for a total of 30 days. The mice were free to drink and feed during the experimental period, and with a 12h/12h day/night cycle. During the experiment, general clinical observation was made after each administration. During administrating, animals were weighed on the day of initiation of administration (Day1), on the first and fourth days of the week, and on the 30th day of administration (Day30); animals were weighed prior to dissection at the endpoint of the experiment. The endpoint of this experiment was the day after the last administration (Day31). At the endpoint of the experiment, mice were dissected for sample collection according to standard procedures. The data were summarized and analyzed for percentage change in body weight, results of anatomical findings, and results of serum assay data. All data were expressed as mean $\pm$ SD, graphed and statistically analyzed using GraphPad Prism 8.0.2 software. For pairwise comparisons, the student's t-test was used. No significance is not shown; shown as $*p < 0.05$, $**p < 0.01$, $***p < 0.001$, and $****p < 0.0001$, respectively, compared to the control group.

The same experimental methods as described above were used for strain MNH06163.

In this experiment, mice in the MNH05119 group were administrated at dosage of approximately $2 \times 10^{10}$ CFU/mouse per administration, and mice in the MNH06163 group were administrated at dosage of approximately $2 \times 10^{10}$ CFU/mouse per administration.

5.2. Experimental results and analysis

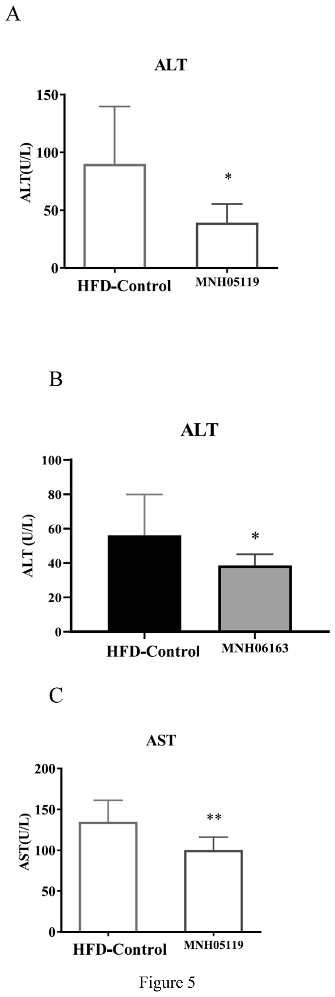

As shown in Figure 5, compared to the HFD-control group, MNH05119 and MNH06163 respectively reduced serum ALT by 56.5% and 31.2%, respectively, and reduced serum AST by 25.7% and 33.2%, respectively. This indicates that MNH05119 and MNH06163 can significantly improve liver function abnormalities induced by a high-fat diet, for example, can treat or prevent liver dysfunction, NAFLD and/or NASH.

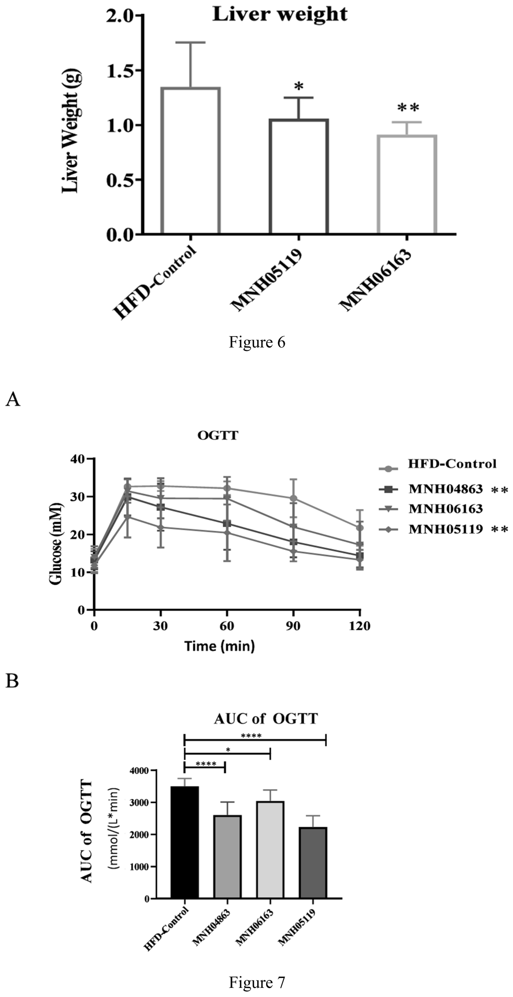

Figure 6 shows that MNH05119 and MNH06163 reduced the liver weight of obesity mice, suggesting that they can reduce the accumulation of liver fat and thus play a role in the treatment of non-alcoholic fatty liver disease (NAFLD and/or NASH).

## Example 6. MNH05119, MNH06163, and MNH04863 for the Prevention or Treatment of Diabetes

6.1 Experiment Methods:

1) Experimental animals: The experimental mice were C57BL/6J mice, aged 5-6 weeks, 18 mice in total, purchased from GuangDong GemPharmatech Co., Ltd.

2) Test strains: The glycerol stocks of the strains MNH05119, MNH04863, and MNH06163 were thawed at 37°C and then inoculated on MM01 plate for activation in an anaerobic workstation. The activated strains were inoculated in MM01 liquid medium and cultured anaerobically to obtain sufficient amount of cultures. These cultures were centrifuged, concentrated and resuspended in a solvent to obtain the subjects with purity and viable bacteria counts ($5\times10^{10}$~$6\times10^{11}$ CFU/mL) that satisfy the requirements of the animal experiments.

3) Negative control (HFD-control group): PBS-Cys was used as a negative control.

4) Experimental procedure: 5 to 6-week-old C57BL/6J male mice were fed with high-fat diet for 10 weeks after quarantine. 36 mice with body weight ranging from 35.50 g-44.49 g were selected and randomly grouped according to their body weights, with 9 mice/group divided into 4 groups (MNH05119, MNH04863, and MNH06163 experimental groups, and the control group). Intragastric administration was started after grouping (Day1), and MNH05119, MNH04863, and MNH06163 were administered in the MNH05119 group, MNH04863 group, and MNH06163 group, respectively, and the negative control substance was administered in the control group. Each group was administered twice daily at a dose of 0.2 mL per administration for a total of 30 days. The mice were free to drink and feed during the experimental period, and with a 12h/12h day/night cycle. During the experiment, general clinical observation was made after each administration. During administrating, animals were weighed on the day of initiation of administration (Day1), on the first and fourth days of the week, and on the 30th day of administration (Day30); animals were weighed prior to dissection at the endpoint of the experiment. Oral glucose tolerance test (OGTT), fasting blood glucose (FBG) and homeostasis model assessment of insulin resistance (HOMA-IR) were measured on the 22nd day (Day22) of administration. The endpoint of this experiment was the day after the last administration (Day31). At the endpoint of the experiment, mice were dissected for sample collection according to standard procedures. The data were summarized and analyzed for percentage change in body weight, results of anatomical findings, results of serum assay data, and HOMA-IR results. All data were expressed as mean $\pm$ SD, graphed and statistically analyzed using GraphPad Prism 8.0.2 software. For pairwise comparisons, the student's t-test was used. For statistical comparisons of more than two groups were analyzed using one-way ANOVA method with Dunnett's multiple comparison test. No significance is not shown; shown as *$p<0.05$, **$p<0.01$, ***$p<0.001$, and ****$p<0.0001$, respectively, compared to the control group.

In this experiment, mice in the MNH04863 group were administrated at dosage of approximately $1\times10^{11}$ CFU/mouse per administration, mice in the MNH05119 group were administrated at dosage of approximately $2\times10^{10}$ CFU/mouse per administration, and mice in the MNH06163 group were administrated at dosage of approximately $2\times10^{10}$ CFU/mouse per administration.

6.2 Oral glucose tolerance test (OGTT)

The Oral Glucose Tolerance Test (OGTT) is a glucose loading test to understand pancreatic β-cell function and the body's ability to regulate blood glucose. Assessing a patient's ability of glucose tolerance is a diagnostic indicator currently recognized for diabetes.

On Day22 of administration, oral glucose tolerance was determined after 12 h of fasting (e.g., fasting from 20:30:00 pm to 08:30:00 pm the following day). Specifically, the fasting body weights of the mice were measured and glucose was administered by gavage at a dose of 2 g/kg (glucose g/mouse fasting body weight kg). Fasting blood glucose and blood glucose values at 15 min, 30 min, 60 min, 90 min, and 120 min post-glucose administration were measured.

The AUC of the OGTT is the area under the curve of the OGTT and is calculated as follows:

$$AUC \text{ of OGTT} = \{(FPG+PG1)*7.5+(PG1+PG2)*7.5+(PG2+PG3)*15+(PG3+PG4)*15+(PG4+PG5)*15\} \; (mmol/(L*min))$$

The results in Figure 7A and 7B showed that after 22 days of treatment, the degree of blood glucose elevation in the high-fat diet-induced obesity mice in the MNH05119-treated group was significantly lower than that in the HFD-control group after 15 min of glucose gavage, which was only 76% of that of the HFD-control group. Additionally, the blood glucose elevation in the MNH05119 group after 15 min of glucose gavage was significantly lower than that in the MNH04863 group, which was only 82% of the MNH04863 group. In the subsequent tests, the blood glucose concentration of the mice in the MNH05119 and MNH06163 treatment groups gradually decreased and the blood glucose values returned to near the initial position after 120 min, which was much lower than that of the HFD-control group, with a significant difference.

Therefore, MNH05119 and MNH06163 can effectively improve the elevation of blood glucose, improve the level of glucose tolerance, and have the effect of preventing or treating diabetes.

MNH04863 has been documented in CN113069475A as having a therapeutic effect comparable to that of the drug liraglutide. Figures 7A and 7B further showed that, unexpectedly, MNH05119 using a much lower concentration dose was able to achieve a more significant reduction effect of oral glucose tolerance in high-fat diet-induced obesity mice than MNH04863. The low-dose MNH06163 group had a comparable reduction effect of oral glucose tolerance in high-fat diet-induced obesity mice compared to that of the high-dose of MNH04863.

6.3 Fasting plasma glucose (FPG) and insulin resistance (HOMA-IR)

At the end of the intervention experiments, mice were fasted overnight for 10 to 12 h. On the following day, the fasting body weights were measured, and blood was collected via orbital puncture under isoflurane anesthesia. The blood samples were kept at 4°C for 3 to 4 h. After the blood coagulated clots contracted, the blood was centrifuged at 4,500 r/min for 15 min at 4°C. Serum was collected from the supernatant. Fasting plasma glucose was measured using a glucometer (ACCU-CHEK, Roche). Insulin levels in serum were detected using a mouse insulin ELISA kit (Wuhan Huamei Bioengineering Co., Ltd.). The insulin resistance index (HOMA-IR) was calculated based on fasting plasma glucose and insulin levels in serum. The calculated formula is:

$$\text{Fasting glucose level (FPG, mmol/L)} \times \text{fasting insulin level (FINS, μU/mL)/22.5.}$$

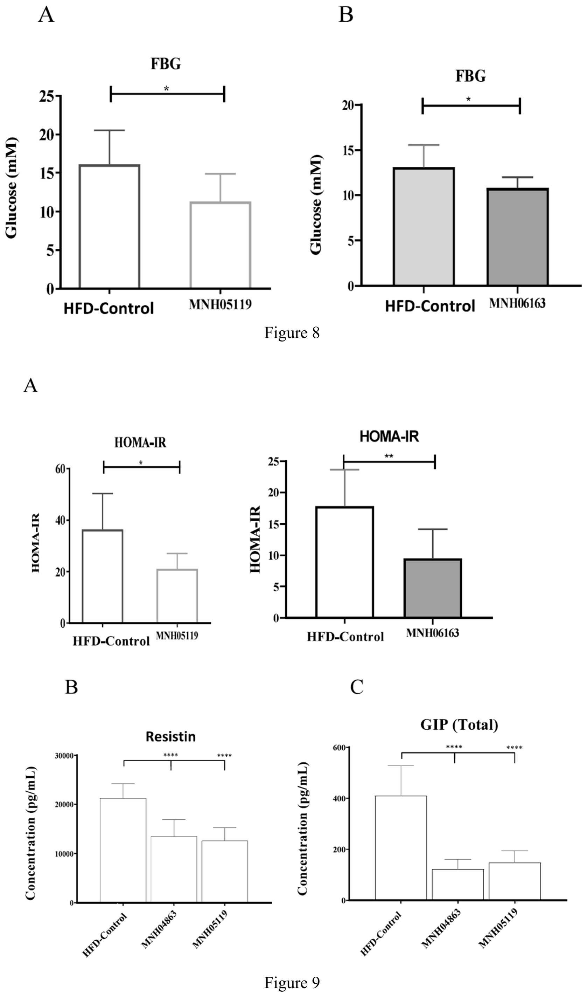

The results in Figure 8 showed that MNH05119 and MNH06163 significantly reduced fasting plasma glucose levels in high-fat diet-induced obesity mice, with reduction percentages of 29.7% and 17.4%, respectively. The results indicated that MNH05119 and MNH06163 had the effect of preventing or treating diabetes.

The results in Figure 9 showed that MNH05119 and MNH06163 significantly reduced HOMA-IR, with reduction percentages of 42.0% and 46.6%, respectively (Figure 9A); MNH04863 and MNH05119 significantly reduced resistin, with reduction percentages of 36.6% and 40.1%, respectively (Figure 9B); and MNH04863 and MNH05119 significantly reduced GIP, with reduction percentages of 36.6% and 40.1%, respectively (Figure 9C). The above experimental data indicated that MNH05119, MNH06163 and MNH04863 could significantly reduce the indicators related to insulin resistance induced by high-fat diet, and have the effect of preventing or treating diabetes.

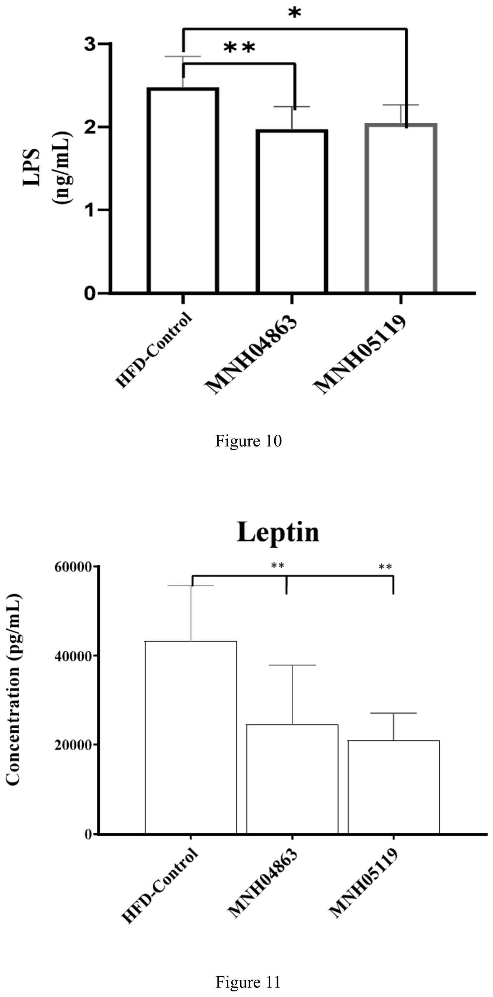

Additional results showed that MNH05119 and MNH04863 could significantly reduce lipopolysaccharide (LPS) in the serum of high-fat diet-induced obesity mice by 17.5% and 20.3%, respectively (Figure 10), suggesting that MNH05119 and MNH04863 could improve endotoxemia, and thus reduce systemic inflammation response. Thus, MNH05119 can increase insulin sensitivity while being able to reduce systemic inflammation and the effect is comparable to those of MNH04863.

Insulin resistance and systemic inflammation are important propelling factors for the occurrence and development of type II diabetes, therefore MNH05119 can achieve the effect of preventing or treating diabetes by improving insulin resistance and reducing systemic inflammation.

6.4 Effects of MNH04863, MNH05119, and MNH06163 on glucagon peptide and leptin resistance in high-fat diet-induced obesity model mice

Figure 11 shows that leptin levels were significantly lower in the MNH04863 and MNH05119 groups (24,536 pg/mL, and 21,021 pg/mL, respectively) compared to that in the HFD-control group (43,244 pg/mL). These results suggest that daily intake of *Christensenella sp.* of the present application can increase the ability of blood glucose regulation, increase leptin sensitivity and improve metabolic disorders caused by obesity.

6.5 Effect of MNH04863 on serum GLP-1, DDP4 (DPPIV) in high-fat diet-induced obesity model mice

On the final day of administration (DAY 30), all animals were fasted overnight for about 12 h. On the following day, blood was collected after isoflurane anesthesia, and 250 μL of blood samples were collected and placed in enzyme inhibitor tubes, which contained 2.5 μL of DPP4 (DPPIV) inhibitor, 2.5 μL of protease inhibitor cocktail, and 7.5 uL of 0.5 M EDTA. Plasma was separated by centrifugation at 4°C, 4500 rpm, for 10 min immediately after blood collection, and the plasma was frozen at ≤ -70°C. Mice plasma GLP-1 levels were assayed using a GLP-1 (Glucagon-Like Peptide-1) (active) ELISA kit.

DPP4 (DPPIV) is an endogenous membrane glycoprotein and serine exopeptidase that cleaves X-proline dipeptides from the N-terminus of polypeptides. DPP4 plays an important role in glucose metabolism through cleavage of incretins such as glucose-dependent insulinotropic ploypeptide (GIP) and glucagon-like peptide-1 (GLP-1) Mouse serum was analyzed for DPP4 (DPPIV) using Luminex technology with the two mouse-magnetic-bead multiplex assay kits, LXSAMSM-23 and LXSAMSM-01.

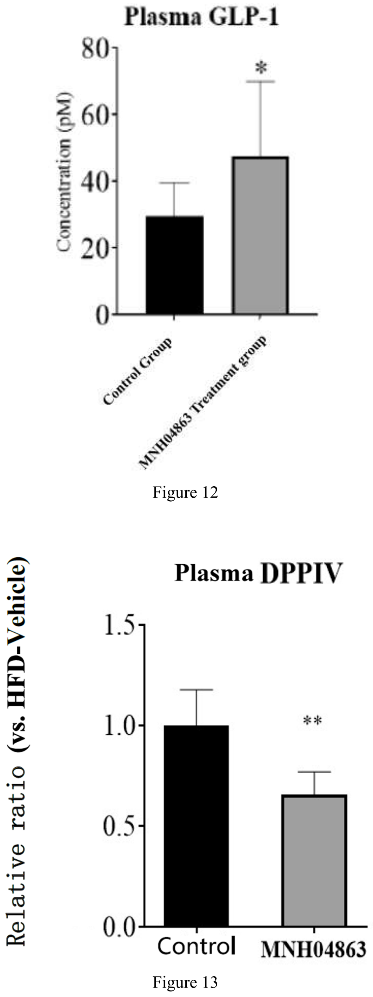

Figure 12 shows that MNH04863 significantly increased GLP-1 concentration in plasma of high-fat diet-induced obesity mice, with a 59.92% elevation compared to HFD-control (HFD-vehicle).

Figure 13 shows that MNH04863 significantly reduced the relative ratio of DPP4 (DPPIV)/CD26 in serum by 34.1% compared to HFD-control (HFD-Vehicle). These results indicate that strain MNH04863 of the present invention can be prepared for use as a DDP4 inhibitor. Thus, it can exert effects of treatment for obesity and diabetes as a DPP4 inhibitor.

## Example 7. Combination of MNH05119 and Metformin for the Treatment of Hyperglycemia in Type II Diabetic Model Mice

7.1 Experiment Methods

(1) Experimental animals: The experimental mice were BKS-Lepr$^{cm2Cd479}$/Gpt (db/db) mice, aged 5-6 weeks, 32 mice in total, purchased from GuangDong GemPharmatech Co., Ltd.

(2) Test strain: The glycerol stock of strain MNH05119 was thawed at 37°C, and then inoculated on MM01 plate for activation in an anaerobic workstation. The activated strain was inoculated in MM01 liquid medium and cultured anaerobically to obtain a sufficient amount of cultures. These cultures were centrifuged, concentrated and resuspended in a solvent to obtain the subjects with purity and viable bacteria counts ($1\times10^{11}$ CFU/mL) that satisfy the requirements of the animal experiments.

3) Negative control (HFD-control group): PBS-Cys was used as a negative control.

4) Positive control group: Metformin, 250 mg/kg, administered by oral gavage.

5) Experimental procedure: 5 to 6-week-old of 32 BKS-Lepr$^{cm2Cd479}$/Gpt(db/db) male mice were randomly grouped according to their body weights after quarantine, with 8 mice/group divided into 4 groups. The groups included the MNH05119 group, the positive control Metformin group, combination administration group (MNH05119+Metformin) and the negative control group. Intragastric administration was started after grouping (Day1), and for each administration, MNH05119 ($2\times10^{10}$CFU/mouse) was administered in the MNH05119 group, metformin (250 mg/kg) was administered in the positive control group, both MNH05119 ($2\times10^{10}$ CFU/mouse) and metformin (250 mg/kg) were administered in the combination administration group, and the negative control substance was administered in the negative control group. MNH05119 was administered twice daily, while metformin and its negative control substance were administered once daily, and the administration route of them were oral gavage, for 28 days. The mice were free to drink and feed during the experimental period, and with a 12h/12h day/night cycle. All groups were dissected for sample collection on the day following final administration (Day29), and the data were summarized to analyze the results of fasting blood glucose, OGTT, and other data. All data were expressed as mean ± SD, graphed and statistically analyzed using GraphPad Prism 8.0.2 software. For comparisons among three or more groups, one-way ANOVA with Dunnett's multiple comparison test was used. No significance is not shown; shown as *$p< 0.05$, **$p< 0.01$, ***$p< 0.001$, and ****$p< 0.0001$, respectively, compared to the control group.

6) Oral Glucose Tolerance Test (OGTT): On the 27th day (Day27) of administration, OGTT was determined after 12 h of fasting (e.g., fasting from 20:30:00pm to 08:30:00pm the following day). The fasting body weights of the mice were measured and glucose was administered by gavage at a dose of 2 g/kg (glucose g/mouse fasting body weight kg). Fasting blood glucose as well as blood glucose values at 15min, 30min, 60min, 90min and 120min post-glucose administration were measured.

7.2 Improvement of oral glucose tolerance in type II diabetic mice by combination of MNH05119 and metformin

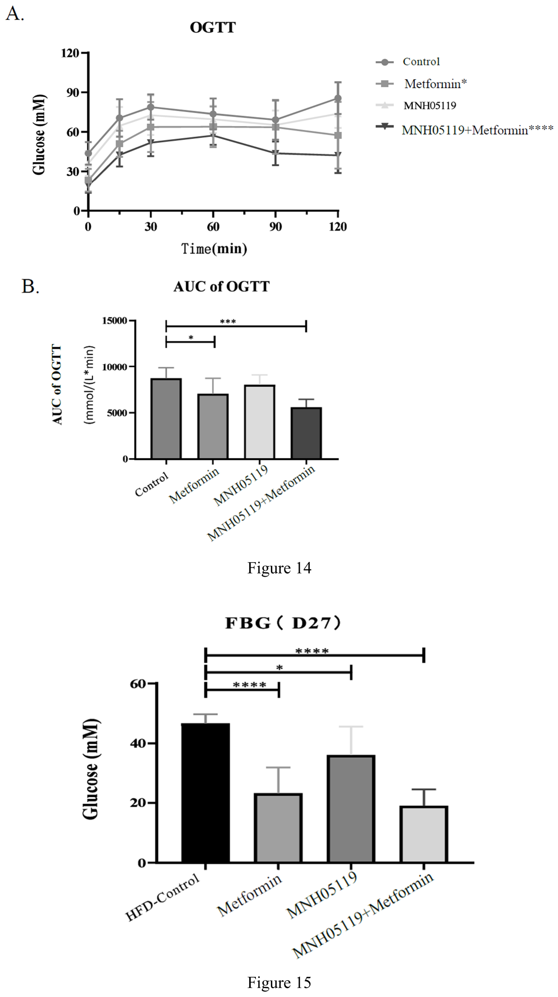

The results in Figure 14 showed that the combination of MNH05119 and metformin more significantly reduced the oral glucose tolerance in type II diabetic mice, in which the AUC-OGTT was reduced by 19.2% in the metformin group, 10.0% in the MNH05119 group, and 35.8% in the combination of MNH05119 and metformin group, compared to the negative control group. These results indicated that the combination of MNH05119 and metformin for the treatment of type II diabetes can achieve a synergistic therapeutic effect in glycemic control.

7.3 Effect of the combination of MNH05119 and metformin on fasting blood glucose in type II diabetic mice

The results in Figure 15 showed that the combination of MNH05119 and metformin more significantly reduced fasting blood glucose in type II diabetic mice, in which compared to the negative control group, blood glucose value was reduced by 50% in the metformin group; 45.5% in the MNH05119 group and 59.1% in the combination of MNH05119 and metformin group. These results indicated that the combination of MNH05119 and metformin for the treatment of type II diabetes can achieve better therapeutic effect in glycemic control.

**Example 8. MNH04863, MNH05119, and MNH06163 for the Treatment and Prevention of Obesity and Related Diseases in a High-Fat Diet-induced Obesity Mouse Model**

8.1 Experiment Methods

1) Experimental animals: The experimental mice were C57BL/6J mice, aged 5-6 weeks, 60 mice in total, purchased from GuangDong GemPharmatech Co., Ltd.
(2) Test strains: The glycerol stocks of strains MNH05119, MNH06163 and MNH04863 were thawed at 37 °C and then inoculated on MM01 plate for activation in an anaerobic workstation. The activated strains were inoculated in MM01 liquid medium and cultured anaerobically to obtain a sufficient amount of cultures. These cultures were centrifuged, concentrated and resuspended in a solvent to obtain the subjects with purity and viable bacteria counts ($5\times10^{10}$~$6\times10^{11}$ CFU/mL) that satisfy the requirements of the animal experiments.
3) Negative control (HFD-control group): PBS-Cys was used as a negative control.
(4) Experimental procedure: 5to 6-week-old C57BL/6J male mice were fed with high-fat diet for 10 weeks after quarantine. 36 mice with body weight ranging from 35.50g-44.49g were selected and randomized according to body weight, 9 mice/group, and divided into 4 groups (MNH05119, MNH06163, MNH04863 and HFD-control group). Intragastric administration was started after grouping (Day1), and for each administration, MNH05119 ($2\times10^{10}$ CFU/mouse), MNH06163 ($2\times10^{10}$ CFU/mouse), and MNH04863 ($1\times10^{11}$ CFU/mouse) were administered in MNH05119 group, MNH06163 group, and MNH04863 group, respectively; the negative control substance was administered in the HFD-control group; and all groups were administered by oral gavage twice daily, 0.2 mL each time, for a total of 30 days. The mice were free to drink and feed during the experimental period, and with a 12h/12h day/night cycle. During the experiment, general clinical observation was made after each administration. During administrating, animals were weighed on the day of initiation of administration (Day1), on the first and fourth days of each week, and on the 30th day of administration (Day30); 24 h intake was measured twice weekly on the second and fifth days; OGTT was measured and animals were weighed on the 22nd day of administration (Day22). At the endpoint of the experiment, the animals were weighed prior to dissection. The endpoint of this experiment was the day after the last administration (Day31). At the endpoint of the experiment, mice were dissected for sample collection according to standard procedures. The data were summarized and analyzed for percentage changes in body weight, results of anatomical findings, and results of serum assay data. All data were expressed as mean ± SD, graphed and statistically analyzed using GraphPad Prism 8.0.2 software. For pairwise comparisons, the student's t-test was used. No significance is not shown; shown as *$p < 0.05$, **$p < 0.01$, ***$p < 0.001$, and ****$p < 0.0001$, respectively, compared to HFD-control group.

8.2 Effect of MNH05119, and MNH06163 on body weight in obesity model mice

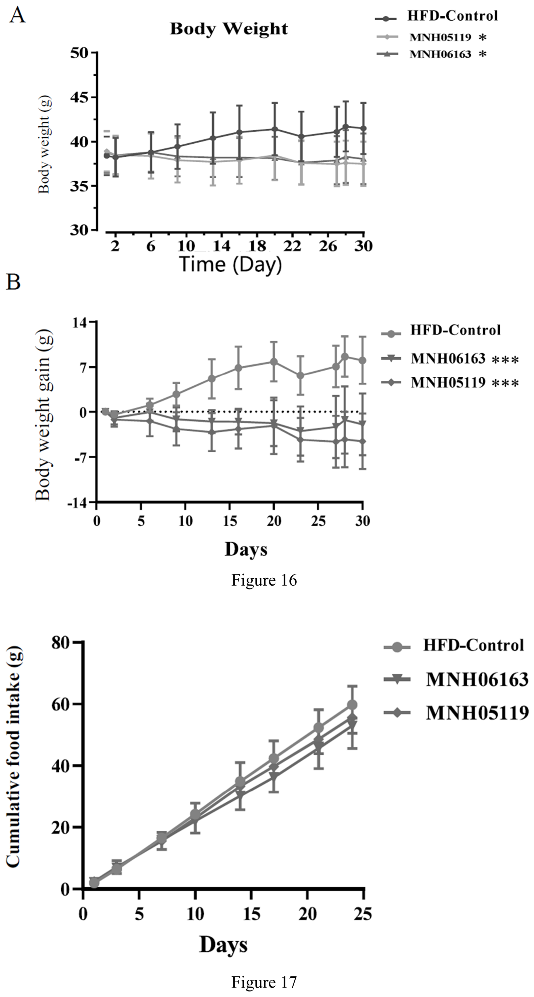

The results in Figure 16 showed that MNH05119 and MNH06163 significantly reduced the body weight (Figure 16A) and body weight gain (Figure 16B) of high-fat diet-induced obesity mice. In the HFD-control group, body weight increased by 8.02%, whereas the body weights of the mice in the MNH06163 group and the MNH05119 group were reduced by 1.96% and 4.56%, respectively, which indicated that MNH05119 and MNH06163 had significant weight-reducing effect compared to the HFD-control group.

8.3 Effects of MNH05119 and MNH06163 on food intake amount of mice in obesity model mice

The results in Figure 17 showed that compared to the HFD-control group, both MNH05119 group and MNH06163 group achieved mouse weight reduction on the basis of increasing mouse energy expenditure without affecting their food intake.

8.4 Effects of MNH05119 and MNH06163 on blood lipids in high-fat diet-induced obesity model mice

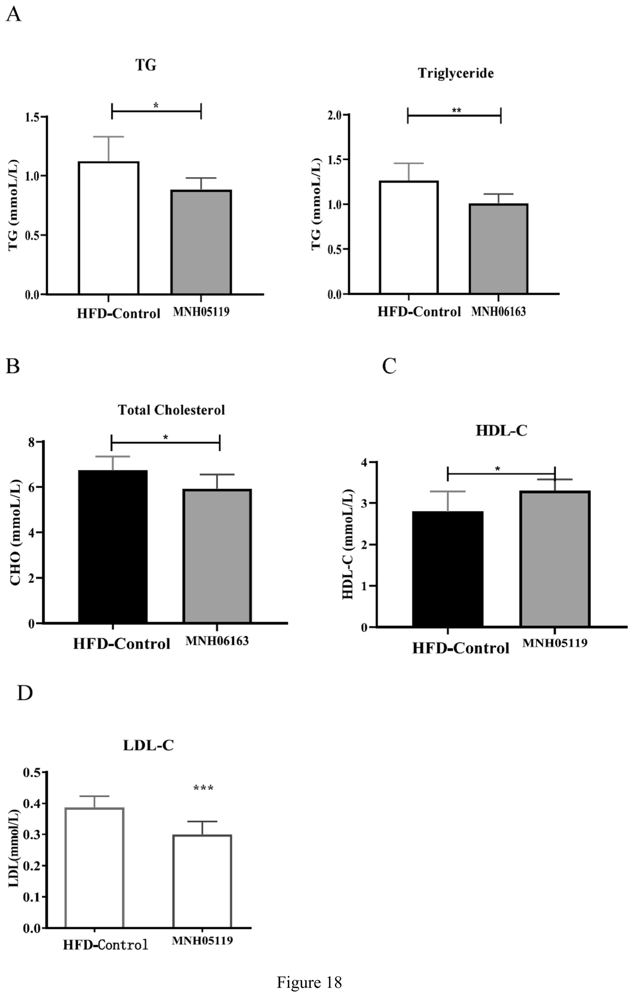

The results in Figure 18A showed that MNH05119 and MNH06163 significantly reduced the serum triglyceride (TG) concentration in high-fat diet-induced obesity mice, indicating that both MNH05119 and MNH06163 had a significant blood lipid-lowering effect, which suggests that they have activity for preventing or treating hyperlipidemia. The results in Figure 18B showed that MNH06163 significantly reduced total cholesterol levels in the serum of high-fat diet-induced obesity mice, indicating a therapeutic/preventive effect on cardiovascular and cerebrovascular diseases. The results in Figure 18C showed that MNH05119 significantly increased the concentration of high-density lipoprotein cholesterol (HDL-C) in high-fat diet-induced obesity mice, indicating the therapeutic/preventive effect of MNH05119 on cardiovascular and cerebrovascular diseases. The results in Figure 18D showed that MNH05119 significantly reduced the low-density lipoprotein cholesterol (LDL-C) content in the serum of high-fat diet mice, indicating a preventive or therapeutic effect on hyperlipidemia and atherosclerosis.

8.5 Effect of MNH05119 on body fat in high-fat diet-induced obesity model mice

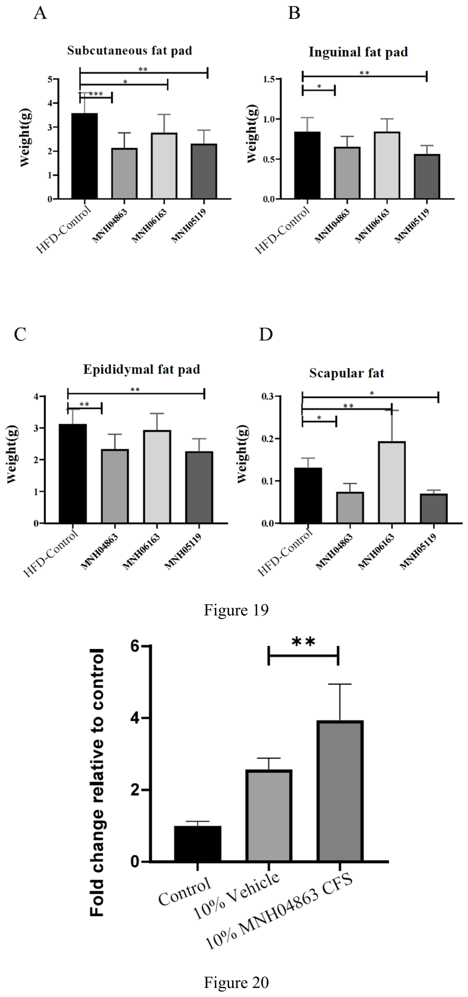

The results in Figure 19A showed that MNH05119 significantly reduced the weight of subcutaneous fat in high-fat diet-induced obesity mice; the results in Figure 19B showed that MNH05119 significantly increased the weight of inguinal fat in high-fat diet-induced obesity mice; and the results in Figure 19C showed that MNH05119 significantly increased the weight of epididymal fat in high-fat diet-induced obesity mice. The results in Figure 19D showed that MNH05119 significantly reduced the scapular fat weight in high-fat diet-induced obesity mice with significant body fat-lowering effect. In conclusion, MNH05119 significantly reduces the weight of adipose tissue and has therapeutic and preventive effects on obesity.

**Example 9. Stimulation of GLP-1 Secretion in NCI-H716 Cells by MNH04863 Extract Product**

NCI-H716 cells (purchased from the Cell Bank of the Chinese Academy of Sciences) were selected for this experiment, and NCI-H716 cells were cultured in RPMI-1640 (Gibco) medium containing 10% fetal bovine serum (Hyclone), 1% double antibiotic (penicillin and streptomycin, Hyclone) in an incubator at 37°C, 5% $CO_2$, where the cells grew in suspension.
Preparation of MNH04863 crude extract: the strain was inoculated into the sterilized MM01 liquid medium with an inoculum of 1% of the total medium volume, static anaerobic culture at 37°C for 24 hours. After two rounds of activation, the fermentation broth was obtained. The broth was centrifuged at 8000rpm for 10min, the concentration of the strain was adjusted to $1 \times 10^{10}$ CFU/mL with a sterile 0.9% NaCl solution, and the strain was cultured anaerobically for 8h. After centrifugation at 8000rpm for 10min, the supernatant was collected for later use.
GLP-1 secretion experiment: NCI-H716 human intestinal L cells were cultured in 96-well cell culture plates coated with Matrigel and cultured for 48h, and the cells were treated for 2h according to the experimental groups; after each group had been treated for 2h, the cell supernatant was collected, and the content of GLP-1 was measured based on the method of GLP-1 Enzyme-Linked Immunosorbent Assay (ELISA) kit. Each treatment group had set six replicates. The data were analyzed by one-way analysis of variance (ANOVA) and Duncan's multiple comparisons using SPSS25.0 software, and the results are shown in Figure 20.
The results showed that 10% crude extract CFS of MNH04863 significantly induced the expression of GLP-1, compared to the control group and the 10% Vehicle group. Glucagon-like peptide (GLP-1) is an incretin hormone secreted by intestinal L-cells, with the important functions such as promoting insulin secretion, inhibiting glucagon secretion, and suppressing gastric emptying and appetite.
Although certain embodiments of the invention have been disclosed herein, it will be apparent that modifications and variations may be made without departing from the spirit and scope of the invention as disclosed herein and as defined by the appended claims. Furthermore, it should be understood that while all examples in the present disclosure illustrate embodiments of the present invention, they are provided solely as non-limiting examples and therefore should not be construed as limiting any aspect of the present invention described herein. The present invention is intended to have the full scope defined by the present disclosure, the language of the following claims, and any equivalents thereof. Accordingly, the drawings and the detailed description should be regarded as illustrative rather than restrictive.

**Claims**

1. An isolated *Christensenella sp.* having a 16S rDNA sequence with at least 98% identity to a sequence shown in SEQ ID NO. 1 or 2; and/or an average nucleotide identity (ANI) value of at least 95% with the strain with a deposit number of GDMCC NO: 62509 or the strain with a deposit number of GDMCC NO: 61118.

2. The *Christensenella sp.* according to claim 1, wherein the average nucleotide identity (ANI) value of the *Christensenella sp.* with the strain with a deposit number of GDMCC NO: 62509 or the strain with a deposit number of GDMCC NO: 61118 is 95.1%, 95.2%, 95.3%, 95.4%, 95.5%, 95.6%, 95.7%, 95.8%, 95.9%, 96%, 96.1%, 96.2%, 96.3%, 96.4%, 96.5%, 96.6%, 96.7%, 96.8%, 96.9%, 97%, 97.1%, 97.2%, 97.3%, 97.4%, 97.5%, 97.6%, 97.7%, 97.8%, 97.9%, 98%, 98.1%, 98.2%, 98.3%, 98.4%, 98.5%, 98.6%, 98.7%, 98.8%, 99%, 99.1%, 99.2%, 99.3%, 99.4%, 99.5%, 99.6%, 99.7%, 99.8%, 99.9% or 100%;
and/or
the 16S rDNA sequence of the *Christensenella sp.* has at least 98%, 98.1%, 98.2%, 98.3%, 98.4%, 98.5%, 98.6%, 98.65%, at least 98.7%, at least 98.8%, at least 98.9%, at least 99.0%, at least 99.1%, at least 99.2%, at least 99.3%, at least 99.4%, at least 99.5%, at least 99.6%, at least 99.7%, at least 99.8%, at least 99.9% or 100% identity to the sequence shown in SEQ ID NO:1 or 2.

3. The *Christensenella sp.* according to claim 1, wherein the *Christensenella sp.* is the *Christensenella sp.* with a deposit number of GDMCC NO: 62509 or GDMCC NO: 61118.

4. A composition comprising the *Christensenella sp.* according to any one of claims 1 to 3, a culture thereof or a metabolite thereof.

5. The composition according to claim 4, wherein the *Christensenella sp.* is a live bacterium, attenuated bacterium, killed bacterium, lyophilized bacterium, or irradiated bacterium; and/or
the culture of the *Christensenella sp.* is a solid culture, fermentation culture, or fermentation culture supernatant.

6. The composition according to claim 4, further comprising one or more pharmaceutically or nutritionally acceptable carriers, excipients, or auxiliaries.

7. The composition according to claim 4, further comprising one or more additional active agents for preventing or treating metabolic diseases, wherein the additional active agent has at least one function selected from the following:

   (a) appetite suppression,
   (b) prevention of metabolic diseases, and
   (c) treatment of metabolic diseases;
   preferably, the additional active agent is selected from: a GLP-1 receptor agonist, a GLP-1 receptor and GCG receptor dual agonist, a GLP-1 receptor, GIP receptor, and GCG receptor triple agonist, an AMPK agonist, or an active drug promoting GLP-1 secretion;
   preferably, the additional active agent is selected from: metformin, sulfonylureas, meglitinides, thiazolidinediones, DPP-4 inhibitor, GLP-1 receptor agonist, SGLT2 inhibitor, insulin, pioglitazone, rosiglitazone, pentoxifylline, Ω-3 fatty acid, statins, ezetimibe, or ursodeoxycholic acid, semaglutide, liraglutide, exenatide, and benaglutide; and
   preferably, the additional active agent is metformin.

8. The composition according to claim 4, wherein the composition is a medicament, health care product, or food product.

9. Use of the *Christensenella sp.* according to any one of claims 1 to 3 or the composition according to any one of claims 4 to 8 in the preparation of a medicament, health care product, or food product for treating, preventing, or alleviating a metabolic disease or a disease caused by a metabolic disorder.

10. The use according to claim 9, wherein the metabolic disease, metabolic disorder, or disease caused by a metabolic disorder is at least one selected from the following: liver disease, obesity and obesity-related disease, cardiovascular disease, diabetes, dyslipidemia, cardiovascular and cerebrovascular disease, glucose intolerance, atherosclerosis, coronary heart disease or hypertension, type I diabetes, type II diabetes, abnormal glucose tolerance, insulin resistance, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic diseases, dyslipidemia, non-alcoholic fatty liver disease (NAFLD), non-alcoholic steatohepatitis (NASH), atherosclerosis, or nephropathy;

preferably, the liver disease is at least one selected from the following: fatty liver, NAFLD/NASH, abnormal liver function, extrahepatic cholestasis, hepatitis, liver injury, intrahepatic cholestasis, liver fibrosis, cirrhosis, and liver cancer; preferably, a trigger of the liver disease is at least one selected from the following: high-fat diet, high-cholesterol diet, high-sugar diet, hyperlipidemia, hyperglycemia, or high cholesterol;

preferably, the obesity and obesity-related disease is at least one selected from the following: overweight, obesity, metabolic syndrome, cardiovascular disease, cardiovascular and cerebrovascular disease, hyperlipidemia, hypercholesterolemia, hypertension, insulin resistance syndrome, obesity-associated gastroesophageal reflux disease, or steatohepatitis; preferably, a trigger of the obesity and obesity-related disease is at least one selected from the following: high-fat diet, high-sugar diet, high cholesterol, hyperlipidemia, hyperglycemia, NAFLD, or NASH;

preferably, the cardiovascular disease or cardiovascular and cerebrovascular disease is at least one selected from the following: atherosclerosis, coronary heart disease, cardiovascular disease in NAFLD or NASH patient, cardiovascular and cerebrovascular disease in NAFLD or NASH patient, or high cholesterol disease; preferably, a trigger of the cardiovascular disease or cardiovascular and cerebrovascular disease is at least one selected from the following: atherosclerosis, NAFLD, NASH, hyperlipidemia, hyperglycemia, or high cholesterol;

preferably, the diabetes is at least one selected from the following: type I diabetes, type II diabetes, gestational diabetes, HDAC activity-mediated diabetes, diabetic nephropathy, diabetic neuropathy, diabetic ophthalmopathy, diabetic retinopathy, diabetic foot, diabetes induced by damage to pancreatic β-cells, diabetes induced by insulin resistance, and diabetes induced by obesity; preferably, a trigger of diabetes includes, but is not limited to, at least one selected from the following: pancreatic islet cell dysfunction, decreased insulin secretion, increased insulin resistance, high-fat diet, high-sugar diet, high cholesterol, hyperlipidemia, hyperglycemia, NAFLD, or NASH; and

preferably, the metabolic disorder is selected from the following: diabetes induced by a disorder of glucose metabolism, diabetes induced by abnormal glucose tolerance or reduced glucose tolerance, diabetes induced by damage to pancreatic β-cells, or diabetes induced by insulin resistance.

11. The use according to claim 9, wherein the medicament, health care product or food product produces at least one effect selected from the following:

reducing liver weight;
treating initial steatohepatitis lesions;
slowing down the accumulation of fat in liver cells;
decreasing serum AST and ALT levels;
reducing inflammatory lesions in abdominal white adipose tissue;
decreasing body weight in mammals;
reducing food intake in mammals;
lowering body fat in mammals;
lowering the levels of at least one indicator selected from the following in mammalian serum: total cholesterol, low-density lipoprotein and triglycerides;
increasing the level of high-density lipoprotein in mammalian serum;
improving impaired oral glucose tolerance in mammals;
lowering fasting blood glucose level in mammals;
lowering HOMA-IR index in mammals;
repairing gastrointestinal mucosal damage;
treating, preventing or alleviating coronary heart disease;
treating, preventing or alleviating atherosclerosis;
treating, preventing or alleviating hyperglycemia;
treating, preventing or alleviating hyperlipidemia;
treating, preventing or alleviating high cholesterol;
treating, preventing or alleviating liver function impairment;
treating, preventing or alleviating fatty liver;
treating, preventing or alleviating NAFLD or NASH;
treating, preventing or alleviating hypertension;
treating, preventing or alleviating diabetes, preferably gestational diabetes, type II diabetes or HDAC activity-mediated diabetes;

treating, preventing or alleviating obesity;

treating, preventing or alleviating metabolic syndrome;

treating, preventing or alleviating localized sebum excess, inguinal fat excess, epididymal fat excess, and/or brown adipose excess.

12. Use of *Christensenella sp.*, a culture thereof, or a metabolite thereof in the preparation of a GLP-1 receptor agonist, wherein the *Christensenella sp.* has one or more characteristics selected from the following:

a 16S rDNA sequence with at least 99% identity to the sequence shown in SEQ ID NO.3; and

the *Christensenella sp.* is Gram-negative;

preferably, the *Christensenella sp.* comprises the strain with a deposit number of GDMCC NO: 61117;

preferably, the culture of the *Christensenella sp.* is a culture supernatant of *Christensenella sp.* or a fermentation supernatant of *Christensenella sp.*;

preferably, the *Christensenella sp.* is *Christensenella intestinihominis*.

13. Use of *Christensenella sp.*, a culture thereof, or a metabolite thereof in the preparation of aDPP4 (DPPIV) inhibitor, wherein the *Christensenella sp.* has a 16S rDNA sequence with at least 99% identity to the sequence shown in SEQ ID NO. 3; and the *Christensenella sp.* is Gram-negative;

preferably, the *Christensenella sp.* comprises the strain with a deposit number of GDMCC NO: 61117;

preferably, extract of the *Christensenella sp.* is a culture supernatant of *Christensenella sp.*, a fermentation supernatant of *Christensenella sp.* or a secondary metabolite of *Christensenella sp.*;

preferably, the *Christensenella sp.* is *Christensenella intestinihominis*.

14. Use of the *Christensenella sp.* according to any one of claims 1 to 3, the composition according to any one of claims 4 to 8, the GLP-1 receptor agonist according to claim 12 or the DPP4 inhibitor according to claim 13 in the preparation of a composition for increasing energy expenditure in a subject by modulating adipose tissue metabolism and/or glucose homeostasis;

preferably, the *Christensenella sp.*, the composition, the GLP-1 receptor agonist or the DPP4 inhibitor does not affect food intake, more preferably does not affect food intake amount, in the subject;

preferably, the composition is a nutritional composition;

preferably, the *Christensenella sp.*, the composition, the GLP-1 receptor agonist or the DPP4 inhibitor is in the form of food additive, dietary supplement, nutraceutical product or medical food.

15. Use of the *Christensenella sp.* according to any one of claims 1 to 3, the composition according to any one of claims 4 to 8, the GLP-1 receptor agonist according to claim 12 or the DPP4 inhibitor according to claim 13 in the preparation of a composition for promoting weight loss in a subject;

preferably, the *Christensenella sp.*, the composition, the GLP-1 receptor agonist or the DPP4 inhibitor does not affect food intake, more preferably does not affect food intake amount, in the subject;

preferably, the composition is a nutritional composition;

preferably, the *Christensenella sp.*, the composition, the GLP-1 receptor agonist or the DPP4 inhibitor is in the form of food additive, dietary supplement, nutraceutical product or medical food.

16. Use of the *Christensenella sp.* according to any one of claims 1 to 3, the composition according to any one of claims 4 to 8, the GLP-1 receptor agonist according to claim 12 or the DPP4 inhibitor according to claim 13 in promoting weight loss in a subject; wherein the *Christensenella sp.* is Gram-negative;

preferably, the *Christensenella sp.*, the composition, the GLP-1 receptor agonist or the DPP4 inhibitor does not affect food intake, more preferably does not affect food intake amount, in the subject;

preferably, the composition is a nutritional composition;

preferably, the *Christensenella sp.*, the composition, the GLP-1 receptor agonist or the DPP4 inhibitor is in the form of food additive, dietary supplement, nutraceutical product or medical food.

17. A *Christensenella sp.*, or a composition comprising *Christensenella sp.*, a culture, or a metabolite thereof for use in increasing energy expenditure in a subject, wherein the *Christensenella sp.* is selected from the *Christensenella sp.* according to any one of claims 1 to 3; and the composition is selected from the composition according to any one of claims 4 to 8;

> preferably, the *Christensenella sp.* or the composition does not affect food intake in the subject, more preferably the *Christensenella sp.* or the composition does not affect food intake amount in the subject;
> preferably, the composition is a nutritional composition;
> preferably, the *Christensenella sp.,* or the composition is in the form of food additive, dietary supplement, nutraceutical product or medical food.

18. The use according to any one of claims 12 to 16, wherein the *Christensenella sp.,* the composition, the GLP-1 receptor agonist or the DPP4 inhibitor is administered in combination with one or more additional probiotics and/or one or more prebiotics.

A

B

Figure 1

A

SU8010 5.0kV 15.1mm x20.0k SE(L) 1/11/2021 09:30    2.00um

B

Figure 2

Figure 3

Figure 4

A

B

C

Figure 5

Figure 6

A

B

Figure 7

A

B

Figure 8

A

B

C

Figure 9

Figure 10

Figure 11

Figure 12

Figure 13

A.

B.

Figure 14

Figure 15

A

B

Figure 16

Figure 17

47

A

B

C

D

Figure 18

A

**Subcutaneous fat pad**

B

**Inguinal fat pad**

C

**Epididymal fat pad**

D

**Scapular fat**

Figure 19

Figure 20

## INTERNATIONAL SEARCH REPORT

| International application No. |
| --- |
| **PCT/CN2024/080883** |

| A. | CLASSIFICATION OF SUBJECT MATTER |
| --- | --- |

C12N1/20(2006.01)i;  A61K35/741(2015.01)i;  A61P3/00(2006.01)i;  A61P 3/10(2006.01)i;  A61P 3/04(2006.01)i

According to International Patent Classification (IPC) or to both national classification and IPC

| B. | FIELDS SEARCHED |
| --- | --- |

Minimum documentation searched (classification system followed by classification symbols)

IPC:C12N A61K A61P

Documentation searched other than minimum documentation to the extent that such documents are included in the fields searched

Electronic data base consulted during the international search (name of data base and, where practicable, search terms used)

CJFD, DWPI; CNABS; CNTXT; CNKI; WOTXT; USTXT; EPTXT; ENTXTC; ENTXT; 万方, WANFANG: 百度学术, Baidu Scholar; BING; Pubmed; ISI web of knowledge; Elsevier Science Direct; Springerlink; NCBI; 中国专利生物序列检索系统, China Patent Biological Sequence Search System: 克里斯滕森, Christensenella, GDMCC, 62509, 61118, 61117, 平均核苷酸同一性, ANI, 16S rDNA, SEQ ID NOs.1-3, GLP-1, 受体激动剂, DPP4, DPPIV, 慕恩(广州)生物科技有限公司, 发明人, inventor, 代谢性疾病, 代谢障碍, 体重, 脂肪, weight, lipid, obesity

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 113069475 A (MOON (GUANGZHOU) BIOTECH CO., LTD.) 06 July 2021 (2021-07-06) <br> abstract, claims 1-10, and description, paragraphs 11-41 and 73-103, and embodiment 1 | 1-2, 4-11, 14-18 |
| Y | CN 113069475 A (MOON (GUANGZHOU) BIOTECH CO., LTD.) 06 July 2021 (2021-07-06) <br> abstract, claims 1-10, and description, paragraphs 11-41 and 73-103, and embodiment 1 | 12-16, 18 |
| Y | CN 115475173 A (ZHEJIANG HUAKANG PHARMACEUTICAL CO., LTD.) 16 December 2022 (2022-12-16) <br> description, paragraphs 11-12, and specific embodiments | 12-16, 18 |
| X | CN 113230387 A (MOON (GUANGZHOU) BIOTECH CO., LTD.) 10 August 2021 (2021-08-10) <br> abstract, and claims 1-10 | 1-2, 4-11, 14-18 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **13 June 2024** | **20 June 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/080883** |

### C.    DOCUMENTS CONSIDERED TO BE RELEVANT

| Category* | Citation of document, with indication, where appropriate, of the relevant passages | Relevant to claim No. |
| --- | --- | --- |
| X | CN 113631174 A (YSOPIA BIOSCIENCES et al.) 09 November 2021 (2021-11-09) abstract, claims 1-22, and description, paragraphs 123-124 | 1-2, 4-11, 14-18 |
| X | WO 2018045493 A1 (SHENZHEN BGI RESEARCH INSTITUTE) 15 March 2018 (2018-03-15) abstract, claims 1-10, and embodiment 1 | 1-2, 4-11, 14-18 |
| X | WO 2022213507 A1 (MOON (GUANGZHOU) BIOTECH CO., LTD.) 13 October 2022 (2022-10-13) abstract, and claims 1-15 | 1-2, 4-11, 14-18 |

Form PCT/ISA/210 (second sheet) (July 2022)

| INTERNATIONAL SEARCH REPORT | International application No. |
|---|---|
| | **PCT/CN2024/080883** |

| **Box No. I** **Nucleotide and/or amino acid sequence(s) (Continuation of item 1.c of the first sheet)** |
|---|

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

International application No.

**PCT/CN2024/080883**

| Box No. II | Observations where certain claims were found unsearchable (Continuation of item 2 of first sheet) |

This international search report has not been established in respect of certain claims under Article 17(2)(a) for the following reasons:

1. [✓] Claims Nos.: **16, 18 (all in part)**
   because they relate to subject matter not required to be searched by this Authority, namely:

   The subject matter of claims 16 and 18 (all in part) relates to a method for treating diseases, which falls within the cases set out in PCT Rule 39.1(iv) for which a search is not required by the International Searching Authority. A search is conducted on the basis that the subject matter thereof is amended to be a corresponding pharmaceutical use.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

Form PCT/ISA/210 (continuation of first sheet) (July 2022)

EP 4 678 732 A1

## INTERNATIONAL SEARCH REPORT
### Information on patent family members

International application No.

**PCT/CN2024/080883**

| Patent document cited in search report | | | Publication date (day/month/year) | Patent family member(s) | | | Publication date (day/month/year) |
|---|---|---|---|---|---|---|---|
| CN | 113069475 | A | 06 July 2021 | None | | | |
| CN | 115475173 | A | 16 December 2022 | None | | | |
| CN | 113230387 | A | 10 August 2021 | None | | | |
| CN | 113631174 | A | 09 November 2021 | CA | 3132239 | A1 | 17 September 2020 |
| | | | | AU | 2020235358 | A1 | 07 October 2021 |
| | | | | SG | 11202109872 | SA | 28 October 2021 |
| | | | | KR | 20210141541 | A | 23 November 2021 |
| WO | 2018045493 | A1 | 15 March 2018 | US | 2019282633 | A1 | 19 September 2019 |
| | | | | US | 10806759 | B2 | 20 October 2020 |
| | | | | JP | 2019517992 | A | 27 June 2019 |
| | | | | JP | 6782302 | B2 | 11 November 2020 |
| | | | | EP | 3511407 | A1 | 17 July 2019 |
| | | | | EP | 3511407 | A4 | 08 April 2020 |
| | | | | EP | 3511407 | B1 | 17 May 2023 |
| WO | 2022213507 | A1 | 13 October 2022 | AU | 2021439233 | A1 | 23 November 2023 |
| | | | | CA | 3214687 | A1 | 13 October 2022 |
| | | | | KR | 20230167089 | A | 07 December 2023 |
| | | | | JP | 2024513071 | A | 21 March 2024 |
| | | | | EP | 4349350 | A1 | 10 April 2024 |

Form PCT/ISA/210 (patent family annex) (July 2022)

54

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 113069475 A

### Non-patent literature cited in the description

- **KIM, M** ; **OH, H.-S.** ; **PARK, S.-C.** ; **CHUN, J**. Towards a taxonomic coherence between average nucleotide identity and 16S rRNA gene sequence similarity for species demarcation of prokaryotes. *International Journal of Systematic and Evolutionary Microbiology*, 2014, vol. 64 (Pt 2), 346-351
- **LIU, C** ; **DU, M.-X.** ; **ABUDUAINI, R** ; **YU, H.-Y.** ; **LI, D.-H.** ; **WANG, Y.-J.** ; **LIU, S.-J.** Enlightening the taxonomy darkness of human gut microbiomes with a cultured biobank. *Microbiome*, 2021, vol. 9 (1), 23
- **JAIN C** ; **RODRIGUEZ-R L M** ; **PHILLIPPY A M et al.** High throughput ANI analysis of 90K prokaryotic genomes reveals clear speciesboundaries[J. *Nature Communications*, 2018, vol. 9 (1), 5114
- **VITAL M** ; **HOWE A C** ; **TIEDJE J M**. Revealing the Bacterial Butyrate Synthesis Pathways by Analyzing (Meta) genomic Data[J. *Mbio*, 2014, vol. 5 (2), 1-11
- **PARK et al.** *J Clin Invest*, 2008, vol. 118, 2316-24
- **CHRISTENSEN et al.** *Mol Med*, 2011, vol. 17 (5-6), 370-390